# NEUE EUROPÄISCHE PATENTSCHRIFT

(11) **EP 2 285 816 B2**
(45) Veröffentlichungstag und Bekanntmachung des Hinweises auf die Entscheidung über den Einspruch: **12.04.2023**
(45) Hinweis auf die Patenterteilung: 01.04.2015
(21) Anmeldenummer: 09757160.8
(22) Anmeldetag: 12.05.2009
(51) Int. Cl.: C07H 1/08, C12N 15/10

(54) **VERFAHREN ZUR ISOLIERUNG VON NUKLEINSÄUREN**
METHOD FOR ISOLATING SHORT CHAIN NUCLEIC ACIDS
PROCÉDÉ D'ISOLATION D'ACIDES NUCLÉIQUES À CHAÎNES COURTES

(30) Priorität: 30.05.2008 EP 08009941
(43) Veröffentlichungstag der Anmeldung: 23.02.2011
(60) Teilanmeldung: 15161652.1
(73) Patentinhaber: Qiagen GmbH, 40724 Hilden (DE)
(72) Erfinder: RITT, Christoph, 40764 Langenfeld (DE); HORLITZ, Martin, 40474 Düsseldorf (DE); SPRENGER-HAUSSELS, Markus, 40822 Mettmann (DE)
(74) Vertreter: König Szynka Tilmann von Renesse Patentanwälte Partnerschaft mbB Düsseldorf
(86) Internationale Anmeldenummer: PCT/EP2009/003364
(87) Internationale Veröffentlichungsnummer: WO 2009/146776

(56) Entgegenhaltungen:
- EP-A- 1 690 938
- WO-A-99/61603
- WO-A2-01/71732
- WO-A2-2005/012487
- US-A1- 2007 106 071
- US-A1- 2007 190 535
- QIAquick(R) Spin Handbook, March 2008 (2008-03),

## Beschreibung

Die vorliegende Erfindung betrifft ein Verfahren zur Isolierung und/oder Aufreinigung von Nukleinsäuren, insbesondere von kurzkettigen Nukleinsäuren.

Die Isolierung von Nukleinsäuren wie DNA und RNA aus pflanzlichen, tierischen oder menschlichen Materialien sowie aus Zellkulturen oder Viruskulturen erfolgt in der Regel nach einem einheitlichen Grundmuster: die Nukleinsäuren enthaltenen Ausgangsmaterialien werden zunächst - teilweise unter Verwendung von proteinabbauenden Enzymen - aufgeschlossen. In nachfolgenden Schritten können die einzelnen Bestandteile unter Verwendung verschiedenster Methoden abgetrennt werden. Darüber hinaus können Nukleinsäuren aus Probenmaterialien isoliert werden, in denen sie frei, d.h. nicht in Zellen, vorliegen. So können freie Nukleinsäuren in künstlichen Probenmischungen vorkommen, jedoch aber auch in natürlichen Proben wie bspw. Blut. Solche frei zirkulierenden Nukleinsäuren werden auch extrazelluläre Nukleinsäuren genannt.

Die meisten der aus dem Stand der Technik bekannten Verfahren zur Aufreinigung der Nukleinsäuren basieren auf einem der beiden folgenden Abtrennungsprinzipien:

Die klassischen Verfahren beruhen auf einem einstufigen Prozess, in dem nach Zusatz eines Puffers, der in den meisten Fällen ein Chaotrop enthält, und nach Zusatz eines organischen Extraktionsmittels - meistens Phenol und/oder Chloroform - eine Extraktion durchgeführt wird. Die unerwünschten Begleitstoffe werden mit der organischen Phase verworfen. Die in der wässrigen Phase verbleibenden Nukleinsäuren können anschießend durch eine Phasenseparation abgetrennt und damit isoliert werden. Der wesentliche Nachteil dieser Verfahrensweise besteht darin, dass neben der Verwendung giftiger und gesundheitsschädigender Stoffe wie Phenol und/oder Chloroform wasserlösliche Stoffe als Verunreinigung in der wässrigen Nukleinsäurelösung verbleiben, die in weiteren, sehr zeitaufwendigen Reinigungsschritten abgetrennt werden müssen.

Angesichts dieser Nachteile hat sich daher im Stand der Technik ein alternativer Prozess durchgesetzt, welcher auf der selektiven Adsorption von Nukleinsäuren an festen Trägermaterialien, wie beispielsweise Siliziumdioxid, basiert. Das nukleinsäurehaltige Ausgangsmaterial wird bei Bedarf lysiert, unter definierten Bedingungen mit dem Trägermaterial in Kontakt gebracht, so dass die Nukleinsäuren an das Trägermaterial binden können; ggf. werden Waschschritte durchgeführt. Optional wird im Anschluss die an den Träger gebundene Nukleinsäure von dem Trägermaterial mittels eines geeigneten Puffers eluiert.

Ein für eine Vielzahl unterschiedlicher Anwendungen geeignetes Verfahren zur Isolierung von Nukleinsäuren ist beispielsweise in der US 5,234,809 (Boom) offenbart. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA bindenden festen Phase beschrieben. Die chaotropen Puffer realisieren, sofern notwendig, sowohl die Lyse des Ausgangsmaterials wie auch die Bindung der Nukleinsäuren an die feste Phase. Das Verfahren ist gut geeignet, um Nukleinsäuren aus kleineren Probemengen zu isolieren. Ein auf einem ähnlichen Prinzip beruhendes Verfahren ist auch in WO93/11221 beschrieben.

EP 1 960 938 offenbart ein Verfahren bei dem Nukleinsäuren in Gegenwart einer chaotropen Verbindung und/oder eines verzweigten oder unverzweigten Alkohols an eine Silikaoberfläche gebunden werden. Die Immobilisierung der Nukleinsäuren erfolgt bei erhöhten Temperaturen. WO99/61603 offenbart ein Plasmidaufreinigungsverfahren, bei dem Nukleinsäuren in Gegenwart von chaotropen Agenzien an ein Silikamaterial gebunden werden. US 2007/106071 betrifft ein Verfahren zur Aufreinigung von Nukleinsäuren unterschiedlicher Länge, bei dem nukleinsäurebindende Phasen mit unterschiedlichen Porengrößen eingesetzt werden.

Im Stand der Technik sind viele Verfahren zur Nukleinsäureaufreinigung bekannt, die aus der Kombination einer festen Phase mit einem chaotropen Puffer bestehen. Da bei allen bekannten Verfahren langkettige Nukleinsäuren mindestens genauso gut, in den meisten Fällen jedoch wesentlich besser an die feste Phase binden als kurzkettige Nukleinsäuren (beispielsweise mit einer Länge von weniger als 1000 bp, 500 bp oder sogar 300 bp), sind die Verfahren aus dem Stand der Technik nicht geeignet, um kurzkettige Nukleinsäuren effizient aufzureinigen bzw. sogar relativ zu den langkettigen Nukleinsäuren anzureichern.

Die schlechte Eignung, kurzkettige Nukleinsäuren aufzureinigen, ist vermutlich darauf zurückzuführen, dass die kurzkettigen Nukleinsäuren schlechter an das Trägermaterial binden, als langkettige Nukleinsäuren (bspw. genomische DNA). Daher gehen kurzkettige Nukleinsäuren bei den meisten der gängigen Aufreinigungsprozessen zu einem großen Teil verloren und finden sich nicht oder unterrepräsentiert unter den aufgereinigten Nukleinsäuren. Für bestimmte Anwendungen ist es jedoch gerade erwünscht, kurzkettige Nukleinsäuren zu isolieren, oder diese gegenüber langkettigen Nukleinsäuren anzureichern.

Um kurzkettige Nukleinsäuren bevorzugt anzureichern bzw. kurzkettige von langkettigen Nukleinsäuren zu trennen, wurden bereits verschiedene Prinzipien im Stand der Technik angewendet. DE 10 2006 045 391 beschreibt beispielsweise ein Verfahren zur Abtrennung langer Nukleinsäuren von kurzen Nukleinsäuren, bei dem die Probe mehrfach über speziell ausgestaltete feste Phasen gegeben wird. Ein anderes Verfahren beruht auf dem Einsatz spezieller Bindepuffer, die keine chaotropen Salze enthalten, sondern Zitronensäuresalze, um so insbesondere kurzkettige Nukleinsäuren zu binden (WO 2007/065934).

Die Aufreinigung/Anreicherung von kurzkettigen Nukleinsäuren (RNA und DNA) ist für verschiedene Anwendungsbereiche von zentraler Bedeutung. Ein Bereich, bei dem kurzkettige Nukleinsäuren eine zentrale Rolle spielen, ist die pränatale Diagnostik. Neben der eigenen frei zirkulierenden DNA befindet sich im Blut schwangerer Frauen zusätzlich noch frei zirkulierende DNA des Fötus. Es wird angenommen, dass sich die frei im Blut schwangerer Frauen zirkulierende fötale DNA von der frei zirkulierenden DNA der Mutter in ihrer Größe unterscheidet. Während die maternale frei zirkulierende DNA oftmals eine durchschnittliche Länge von über 500 bp besitzt, ist ein überwiegender Teil der fötalen frei zirkulierenden DNA deutlich kleiner und besitzt im Schnitt eine Länge von unter 500 bp. Dieser Größenunterschied zwischen den fötalen und den maternalen Nukleinsäuren könnte ausgenutzt werden, um fötale DNA anzureichern und damit besser untersuchen zu können, sofern geeignete Aufreinigungsmethoden zur Verfügung stünden. Die Nutzung der frei zirkulierenden DNA fötalen Ursprungs zur pränatalen Diagnostik hätte den Vorteil, dass es gegenüber klassischen Methoden wie die Amniozentese oder der Chorionzottenbiopsie für den Fötus ungefährlich wäre und daher weniger Risiken bergen würde. Jedoch sind die Mengen an frei zirkulierender DNA fötalen Ursprungs im Blut sehr gering. Je nach Stadium der Schwangerschaft befinden sich in 1 ml Blut zwischen 20 und 260 Kopien fötaler DNA. Die Konzentration an frei zirkulierender fötaler DNA ist damit zwar gering, aber immer noch höher als die Konzentration an frei zirkulierenden fötalen Zellen. Ferner besteht das Problem, dass die Konzentration der frei zirkulierenden fötalen DNA im Vergleich zur frei zirkulierenden maternalen DNA äußerst gering ist, so dass bei der Gesamtisolation frei zirkulierender DNA aus maternalem Blut nur ein Bruchteil der Erbsubstanz vom Fötus stammt; der überwiegende Teil der isolierten Erbsubstanz stammt von der Mutter. Durch den hohen Hintergrund an maternaler DNA ist in vielen Fällen die Detektion von Genabschnitten des Fötus erschwert, in manchen Fällen reicht die Sensitivität selbst so empfindlicher Nachweismethoden wie der Real-Time PCR nicht aus, um die fötale DNA nachweisen zu können.

Die bislang eingesetzten Methoden zur Isolation von frei zirkulierender DNA aus maternalem Blut reinigen oftmals im gleichen Maße die fötale wie die maternale DNA auf, so dass das ungünstige Verhältnis von fötaler zu maternaler DNA bestehen bleibt - die fötale DNA macht nur einen Bruchteil der Gesamt-DNA aus. Eine Anreicherung von fötaler DNA gegenüber der maternalen DNA erfolgt bislang nicht. Ferner werden die fötalen Nukleinsäuren sogar regelmäßig schlechter aufgereinigt, da kurzkettige Nukleinsäuren oftmals weniger gut bei der Aufreinigung erfasst werden. Da sie bei der Aufreinigung schlechter erfasst werden, liegen sie dann in der aufgereinigten Probe im Verhältnis in geringerer Konzentration vor als in der Ausgangsprobe. Eine effiziente Aufreinigung oder sogar spezifische Anreicherung der kurzkettigen extrazellulären Nukleinsäuren wie bspw. der fötalen DNA wäre jedoch vorteilhaft, weil damit die Sensitivität und damit die Verlässlichkeit einer pränatalen Diagnostik, die auf frei zirkulierender fötaler DNA basiert, wesentlich erhöht würde.

Der vorliegenden Erfindung liegt daher die Aufgabe zugrunde, ein Verfahren zur Isolierung und/oder Aufreinigung von Nukleinsäuren zur Verfügung zu stellen, mit dem sich extrazelluläre Nukleinsäuren und insbesondere auch kurzkettige Nukleinsäuren effizient isolieren/aufreinigen lassen. Ferner ist es Aufgabe der Erfindung, ein Verfahren zur Isolation von fötaler DNA aus maternalem Blut bereit zu stellen, das eine effektive Isolierung und/oder Anreicherung der fötalen DNA erlaubt.

Die Aufgabe wird vorliegend gelöst durch ein Verfahren zur Isolierung und/oder Aufreinigung von kurzkettigen Nukleinsäurenmit einer Länge von ≤ 500bp, aus einem nukleinsäurehaltigen Ausgangsmaterial, wie in Anspruch 1 definiert.

Nach dem erfindungsgemäßen Verfahren gemäß Anspruch 1 wird die Immobilisierung der zu isolierenden Nukleinsäuren an das beanspruchte Trägermaterial unter spezifischen Reaktionsbedingungen vorgenommen. Entscheidend für die effektive Bindung der kurzkettigen Nukleinsäuren an das Trägermaterial sind zwei Komponenten, nämlich wenigstens eine chaotrope Verbindung sowie ein verzweigter und/oder unverzweigter Alkohol, wobei es sich gemäß Anspruch 1 bei der chaotropen Verbindung um Guanidiniumthiocyanat oder Guanidiniumisothiocyanat und bei dem Alkohol um Isopropanol handelt. Besonders wichtig ist erfindungsgemäß die richtige Wahl der Alkoholkonzentration, die im Probengemisch bei der Bindung an das Trägermaterial ≥ 15% (v/v) ist. Bevorzugt liegt die Alkoholkonzentration jedoch höher und daher bei ≥ 19% (v/v). Es hat sich gezeigt, dass bei der Wahl der richtigen Alkoholkonzentration kurzkettige Nukleinsäuren sehr gut an das Trägermaterial binden. Eine niedrigere Alkoholkonzentration kann in gewissem Umfang durch eine Erhöhung der Konzentration an chaotropen Substanzen ausgeglichen werden. Es besteht daher eine Wechselwirkung zwischen der Alkoholkonzentration und der Konzentration an chaotropen Verbindungen, auf die im Folgenden nach eingegangen werden wird. Die erfindungsgemäße Einstellung der Konzentration des Alkohols bewirkt, dass insbesondere kurzkettige Nukleinsäuren effizient gebunden und damit bei der Aufreinigung erfasst werden.

Gemäß einer Ausführungsform liegt die Alkoholkonzentration in einem Bereich von ca. 19 bis 25% (v/v), da in diesem Bereich kurzkettige Nukleinsäuren und insbesondere DNA besonders gut gebunden und damit isoliert werden können. Auch die Konzentration an chaotropen Verbindungen beeinflusst die Bindung der Nukleinsäuren. Bei Wahl einer höheren Konzentration an chaotropen Verbindungen können auch geringere Alkoholkonzentrationen eingesetzt werden.

Die Alkoholkonzentration liegt in Schritt (a) in einem Bereich von ≥ 15% und ≤ 25% (v/v). Gemäß Anspruch 1 handelt es sich bei dem Alkohol um Isopropanol. In Schritt (a) kann der Alkohol in der Mischung bspw. in einer Konzentration von ca. 18 bis 20% (v/v) vorliegen. Versuche haben gezeigt, dass auch bei diesen niedrigen Alkoholkonzentrationen unterschiedlich lange Nukleinsäuren und auch kurzkettige Nukleinsäuren effizient aufgereinigt werden können und damit ein breites Größenspektrum effektiv erfasst wird. Um eine gute Erfassung der kurzkettigen Nukleinsäuren zu gewährleisten, sollte die chaotrope Verbindung in Schritt (a) in einer ausreichend hohen Konzentration vorliegen. Die Konzentration der chaotropen Verbindung in Schritt a) liegt daher bei ≥ 2 mol/l. Als chaotrope Verbindungen werden eingesetzt Guanidiniumthiocyanat oder Guanidiniumisothiocyanat. Diese stark chaotrope Verbindungen werden in Kombination mit niedrigeren Alkoholkonzentrationen von ≤ 25% (v/v) eingesetzt. Die Konzentrationen liegen bei ≥ 2,0 mol/l und ≤ 3,1 mol/l.

Optional können Waschschritte durchgeführt werden. Die an das Trägermaterial gebundenen Nukleinsäuren werden dann vom Trägermaterial abgetrennt, bspw. in an sich bekannter Weise eluiert, sofern eine Gewinnung der kurzkettigen Nukleinsäuren gewünscht ist. Die erfindungsgemäß isolierten/angereicherten Nukleinsäuren können dann in bekannter Weise weiterverarbeitet, also bspw. analysiert werden. Je nach geplanter anschließender Weiterverarbeitung oder Analyse ist es jedoch ebenfalls möglich, die Nukleinsäuren gebunden am Trägermaterial und damit ohne Elution einzusetzen. Ferner kann das Verfahren dazu eingesetzt werden, Nukleinsäuren aus einer Probe zu entfernen.

Das erfindungsgemäße Verfahren ist als 1-Schritt Verfahren durchführbar. Der Vorteil eines 1-Schritt Verfahrens liegt darin, dass in der Regel eine höhere Ausbeute an Nukleinsäuren (insgesamt) erzielt wird; Nukleinsäuren gehen während der Aufreinigung nur in einem geringen Maß verloren. Da mit dem erfindungsgemäßen Verfahren in der 1-Schritt Variante die kurzkettigen Nukleinsäuren effektiv an das Trägermaterial gebunden und damit isoliert/angereichert werden können, ist die so erfolgte Isolierung/Anreicherung der kurzkettigen Nukleinsäuren für viele Anwendungen bereits ausreichend. Ein 1-Schritt Verfahren ist für den Anwender auch besonders angenehm, da es schnell und einfach durchführbar ist. Wie die in den Beispielen gezeigten Vergleichsversuche belegen, ist das erfindungsgemäße 1-Schritt Verfahren zur Isolierung kurzkettiger Nukleinsäuren den im Stand der Technik bekannten Verfahren deutlich überlegen, weil mit dem erfindungsgemäßen Verfahren eine effektive Isolierung kurzkettiger Nukleinsäuren mit guter Ausbeute und teilweise sogar auch eine Anreicherung der kurzkettigen Nukleinsäuren gegenüber den langkettigen Nukleinsäuren erzielt werden kann.

Für bestimmte offenbarte Anwendungen ist es vorteilhaft, eine möglichst effiziente Anreicherung der kurzkettigen Nukleinsäuren gegenüber den langkettigen Nukleinsäuren zu erzielen; die kurzkettigen Nukleinsäuren sollen ohne bzw. mit möglichst wenig Hintergrund an langkettigen Nukleinsäuren gewonnen werden. In diesem Fall ist es wünschenswert, so wenig langkettige Nukleinsäuren wie möglich mit aufzureinigen. Um dies besonders effektiv zu erreichen, wird gemäß einer Ausführungsform des erfindungsgemäßen Verfahrens den eigentlichen Isolationsschritten a) und optional b) zur Abtrennung/Elution der kurzkettigen Nukleinsäuren ein Schritt (x) vorgelagert, durch den eine effiziente Abreicherung der langkettigen Nukleinsäuren erzielt wird. Durch die vorherige Abreicherung der langkettigen Nukleinsäuren können kurzkettige Nukleinsäuren in besonders reiner Form gewonnen werden.

Gemäß dieser offenbarten 2-Schritt Variante des erfindungsgemäßen Verfahrens ist den eigentlichen Isolationsschritten a) und b) zur Isolation/Aufreinigung der kurzkettigen Nukleinsäuren der Schritt (x) vorgelagert, durch den eine effiziente Abreicherung der langkettigen Nukleinsäuren erzielt wird. Im Bindungsschritt (x) erfolgt die Bindung der Nukleinsäuren an das Trägermaterial in Gegenwart von chaotropen Verbindungen und einem verzweigten und/oder unverzweigten Alkohol. Entscheidend für die effiziente Abreicherung der langkettigen Nukleinsäuren sind wiederum die Bindungsbedingungen und insbesondere die Konzentration des während der Bindung in der Gesamtprobe vorhandenen Alkohols. Aus dem insbesondere die kurzkettigen Nukleinsäuren enthaltenden Durchbruch/Überstand aus Schritt (x) werden dann in den sich anschließenden Schritten a) und optional b) die kurzkettigen Nukleinsäuren isoliert und damit angereichert.

Gemäß einer Ausführungsform sind die Schritte a) und b) des 1-Schrittverfahrens (siehe oben) den Schritten a) und b) des 2-Schritt Verfahrens sehr ähnlich bzw. sind sie nahezu identisch. Beim 2-Schrittverfahren wird letztlich anstelle des nukleinsäurehaltigen Ausgangsmaterials der Durchbruch/Überstand aus Schritt (x) eingesetzt. Es gelten daher für die Schritte a) und b) des 2-Schrittverfahrens die gleichen bevorzugten Bedingungen wie im Zusammenhang mit dem 1-Schrittverfahren beschrieben. Durch den vorgelagerten Schritt (x) erfolgt letztlich zusätzlich eine Abreicherung der langkettigen Nukleinsäuren, so dass sich diese zumindest in geringerer Menge in dem Durchbruch/Überstand befinden. Sofern gewünscht, können im Falle des 2-Schrittverfahrens die langkettigen Nukleinsäuren ebenfalls von dem in Schritt (x) gebundenen Trägermaterial eluiert und für andere Zwecke weiterverwendet werden.

Wie ausgeführt hat die Art und Konzentration der chaotropen Verbindung einen Einfluss auf die Bindung der Nukleinsäuren und insbesondere auf die Effizienz der Bindung der kurzkettigen Nukleinsäuren. Sofern die Konzentration an Alkohol daher im vorgelagerten Schritt (x) und im sich anschließenden Schritt (a) daher gleich oder nahezu gleich ist, wird in Schritt (x) entweder eine geringere Konzentration an chaotropen Verbindungen und/oder eine schwächer chaotrope Verbindung eingesetzt als in Schritt (a). Hierdurch läßt sich wiederum eine Abreicherung der langkettigen und damit eine Anreicherung der kurzkettigen Nukleinsäuren erzielen. Ein geeignetes Beispiel für eine schwächer chaotrope Verbindung, die in Schritt (x) eingesetzt werden kann ist bspw. Guanidiniumhydrochlorid.

Gemäß der vorliegenden Offenbarung können verschiedene Materialien und insbesondere biologische Materialen können als nukleinsäurehaltige Ausgangsmaterialien zum Einsatz kommen. Hierzu gehören bspw. Viren, Phagen und Zellen, wie z. B. Bakterien aber auch humane, tierische oder pflanzliche Zellen. Daneben eignet sich das Verfahren jedoch insbesondere auch zur Isolierung/Aufreinigung von freien Nukleinsäuren aus nichtzellhaltigen Proben, oder entsprechend aufbereiteten Proben. Insbesondere eignet sich das offenbarte Verfahren bspw. für die Isolierung von Nukleinsäuren wie DNA und/oder RNA aus Probenmaterialen humanen oder tierischen Ursprungs, insbesondere klinischen Proben, wie Blut, Plasma, Serum, Mundspülflüssigkeit, Urin, Zerebralflüssigkeit, Sputum, Stuhl, Punktate, Epithelabstriche, Biopsien und anderen Geweben oder Knochenmarkproben. Gemäß Anspruch 1 ist das erfindungsgemäße Verfahren zur Isolierung und/oder Aufreinigung von kurzkettiger, frei zirkulierender DNA aus Plasma oder Serum. Insbesondere ist das erfindungsgemäße Verfahren zur Isolierung von fötaler DNA aus maternalen Blutproben und insbesondere Blutplasma geeignet. Ebenso ist das erfindungsgemäße Verfahren insbesondere für die Isolierung frei zirkulierender Nukleinsäuren wie beispielsweise Tumor-DNA und -RNA aus Körperflüssigkeiten wie insbesondere Plasma und/oder Serum geeignet.

In bestimmten Fällen kann die Probe ohne Vorbehandlung in dem erfindungsgemäßen Verfahren eingesetzt werden. In vielen Fällen muss die Probe jedoch zunächst durch eine geeignete Methode aufgeschlossen und das in der Probe enthaltene biologische Material freigesetzt werden. Verfahren zum Aufschluss von Proben und Zellen sind dem Fachmann bekannt und können chemischer, enzymatischer oder physikalischer Natur sein. Auch eine Kombination dieser Verfahren ist möglich.

Hierbei können sich verschiedene Faktoren für unterschiedliche biologische Materialien als vorteilhaft herausstellen; prinzipiell sind folgende Methoden gut geeignet: Lyse mit Hilfe von ionischen und nicht-ionogenen Detergenzien, wie z. B. SDS, LiDS oder Sarkosyl in geeigneten Puffern, der Einsatz von chaotropen Salzen, wie beispielsweise Guanidiniumhydrochlorid (GHCL), Guanidiniumthiozyanat (GTC), Guanidiniumisothiozyanat (GITC), Natriumiodid, Natriumperchlorat u. a.; mechanisches Auseinanderreißen, wie z. B. mittels einer French Press, Ultraschall, Mahlen mit Glaskugeln, Nickelkugeln, Aluminium oder in flüssigen Stickstoff; enzymatische Lyse, beispielsweise mit Lysozym, Proteinasen, Proteinase K oder Zellulasen oder mittels anderen kommerziell erhältlichen Enzymen für die Lyse; Lyse der Zellen mittels Bakteriophagen oder Virusinfektionen; Gefriertrocknen; osmotischer Schock; Mikrowellenbehandlung; Temperaturbehandlung; beispielsweise Erwärmen oder Kochen oder Gefrieren, z. B. in Trockeneis oder flüssigem Stickstoff und Auftauen; alkalische Lyse.

Wie ausgeführt, stellen die vorstehenden Verfahren Stand der Technik zur Lyse da, die hinlänglich bekannt sind und daher nicht im Detail erläutert werden müssen.

Bei der Aufreinigung von frei zirkulierender DNA aus einer Blutprobe, wie bspw. zur Aufreinigung fötaler DNA aus einer maternalen Probe, werden zunächst die Zellen und weitere feste Bestandteile des Blutes abgetrennt (bspw. durch Zentrifugation), und das so gewonnene Plasma weiterverarbeitet. Das Plasma ist üblicherweise frei von Zellen und enthält die frei zirkulierende Nukleinsäure, bspw. maternale und fötale DNA. Bei der Aufreinigung von freien Nukleinsäuren aus Plasma ist keine eigentliche Zelllyse erforderlich, um die Nukleinsäuren freizusetzen, da diese schon in frei zirkulierender Form vorliegen. Dies gilt auch für andere Proben, bei denen die Nukleinsäuren frei vorliegen und sich entsprechend nicht in Zellen befinden. Frei zirkulierende Nukleinsäuren können jedoch komplexiert mit Proteinen und/oder anderen Stoffen vorliegen. Aus diesem Grunde wird das nukleinsäurehaltige Ausgangsmaterial, beispielsweise das Blutplasma, zunächst mit einem Release-Puffer behandelt, der sicherstellt, dass die Nukleinsäuren aus der komplexierten Form freigesetzt werden. Der Release-Puffer ist in seiner Funktion und Zusammensetzung ähnlich einem Lyse-Puffer, der zum Zellaufschluss eingesetzt wird; durch den Release-Puffer werden in der Probe geeignete Bedingungen für die Freisetzung der Nukleinsäuren erzeugt, so dass diese nicht komplexiert vorliegen. Durch die Zugabe des Release-Puffers wird die Nukleinsäure der Aufreinigung besser zugänglich. Das erfindungsgemäße Verfahren kann entsprechend auch bei zellfreien Ausgangsmaterialien eingesetzt werden. Der entsprechende Release-Puffer kann, je nach Zusammensetzung, auch als Lyse-Puffer fungieren, um Nukleinsäuren effektiv aufzureinigen bzw. anzureichern. In der Regel können die üblichen Lyse-Puffer auch als Release-Puffer eingesetzt werden.

Erfindungsgemäß ist der Einsatz von chaotrophaltigen Release - bzw. Lyse-Puffern besonders effektiv. Dies insbesondere, da auch die Zusammensetzung des Release- bzw. Lyse-Puffers die Bedingungen beeinflusst, unter denen die Nukleinsäuren an das Trägermaterial binden. Die Bindungsbedingungen in der Probe, die erfindungsgemäß entscheidend für die Effektivität des erfindungsgemäßen Verfahrens sind, können daher auch durch geeignete Wahl des Release- bzw. Lyse-Puffers bspw. in Kombination mit einem Bindepuffer eingestellt werden. Release- bzw. Lysepuffer gemäß der vorliegenden Erfindung enthalten eine chaotrope Verbindung, wie z. B. GTC oder GHCL und ggf. ein Detergenz, wie z. B. SDS oder Tween. Diese Agenzien können in wässriger Lösung oder in einer Pufferlösung, d. h. als sog. Release- oder Lysepuffer vorliegen. Als Puffer kann jeder geeigneter Puffer, wie beispielsweise Tris, Bicin, Tricin oder Phosphatpuffer eingesetzt werden. Alternativ kann das Lyse- bzw. Release Agens auch getrennt zugegeben werden. Geeignete Konzentrationen und Mengen der Lyse- bzw. Release Agenzien variieren in Abhängigkeit von den betreffenden Systemen, Art der Zellen etc. und können durch den Fachmann bestimmt werden. Für spezifische Anwendungen, insbesondere die Aufreinigung von fötaler DNA aus Blutproben haben sich beispielsweise Konzentrationen im Bereich von 2 bis 7 M chaotrope Verbindungen, wie z. B. GTC, GHCL oder Nal oder Natriumperchlorat, 0,1 M bis 1 M alkalische Agenzien, wie z. B. NaOH sowie 0,1 bis 50 Gew% (w/v) Detergenzien, wie insbesondere nicht-ionische Detergenzien wie beispielsweise Tween, bewährt.

Chaotrope Verbindungen wie in Anspruch 1 definiert sind auch während der Bindung der Nukleinsäuren an das Trägermaterial in dem Probengemisch vorhanden. Die chaotropen Verbindungen können bspw. aus dem Lyse- bzw. Release-Puffer stammen und/oder aber separat bspw. in Form eines Bindepuffers hinzugegeben werden. Entscheidend sind letztlich die Bindungsbedingungen in der Probe während der Bindung der Nukleinsäuren an das Trägermaterial. Der Einsatz chaotroper Verbindungen ist vorteilhaft für die effiziente Bindung der Nukleinsäure. Die Konzentration der beanspruchten chaotropen Verbindungen in der Probe liegt während der Bindung in einem Bereich wie in Anspruch 1 definiert. Die Wechselwirkung der Konzentration und Art der eingesetzten chaotropen Verbindung und der Alkoholkonzentration bei der Einstellung der Bindungsbedingungen wurde oben bereits im Detail diskutiert. Wir verweisen auf die obige Offenbarung.

Ferner weist die Probe während der Bindung nicht-ionische Detergenzien und insbesondere Tween auf. Die Detergenzien können entweder mit dem Release/Lysepuffer zugegeben werden, oder Bestandteil des Bindepuffers sein. Detergenzien bewirken eine effiziente Solubilisierung verschiedener Bestandteile in der Probe gemäß Anspruch 1, so bspw. von Serum- und Plasmabestandteilen. Dadurch kann eine Verstopfung des nukleinsäurebindenden Trägermaterials verhindert werden. Dies ist insbesondere bei Einsatz einer Silikamembran von Vorteil.

Wie ausgeführt, ist die Konzentration des Alkohols in der Probe während der Bindung der Nukleinsäuren an das Trägermaterial bei dem erfindungsgemäßen Verfahren entscheidend. Offenbartwerden kurzkettige verzweigte oder unverzweigte Alkanole mit einem bis fünf Kohlenstoffatomen, wie z. B. Methanol, Ethanol, Propanol, Isopropanol, Butanole oder Pentanole. Auch Mischungen der entsprechenden Alkohole werden offenbart. Gemäß Anspruch 1 wird zur Bindung Isopropanol eingesetzt.

Sofern in einem Vorschritt (x) die langkettigen Nukleinsäuren abgereicht werden, kommen Alkoholkonzentrationen zum Einsatz, die die Bindung der langkettigen Nukleinsäuren an das Trägermaterial fördern, um diese dadurch aus der Probe entfernen zu können. Die Wechselwirkung der Konzentration an chaotroper Verbindung und der Alkoholkonzentration wurde oben bereits im Detail diskutiert. Wir verweisen auf die obige Offenbarung. Der Durchbruch bzw. der Überstand enthält die gewünschten kurzkettigen Nukleinsäuren aufgrund der Abreicherung zu einem größeren Anteil.

Im Schritt a) des offenbarten 2-Schritt Verfahrens wird eine höhere Alkoholkonzentration zur bevorzugten Bindung der kurzkettigen Nukleinsäuren eingesetzt. Zur besseren Bindung der kurzkettigen Nukleinsäuren ist die Alkoholkonzentration in der Probe während der Bindung ≥ 15% (v/v), ≥ 19% (v/v). Bei Einsatz einer geringeren Alkoholkonzentration von ≤ 25% (v/v) in Schritt (a) wird gemäß Anspruch 1 eine stärker chaotrope Verbindung, nämlich Guanidinumisothiocyanat oder Guanidiniumthiocyanat, eingesetzt. Dieses Ergebnis ist insbesondere für die Aufreinigung von fötaler DNA aus Blutproben vorteilhaft, da die Proben wie bspw. maternales Blutplasma normalerweise überwiegend langkettige Nukleinsäuren enthalten. Mit dem erfindungsgemäßen Verfahren kann daher sogar eine effektivere Aufreinigung der kurzkettigen Nukleinsäuren erzielt werden.

Beim offenbarten 2-Schrittverfahren, bei dem in Schritt (x) eine Abreicherung der langkettigen Nukleinsäuren erfolgt, ist die Konzentration der chaotropen Verbindungen in Schritt a) ≥ der Konzentration an chaotropen Verbindungen in Schritt (x). Es werden deutlich bessere Ergebnisse erzielt, wenn die Konzentration der chaotropen Verbindungen beim Übergang des Verfahrensschritt (x) zum dem Verfahrensschritt a) nicht gesenkt wird, sondern die Alkoholkonzentration erhöht wird. Bevorzugt wird im eigentlichen Isolationsschritt a) sogar eine höhere Konzentration an chaotropen Verbindungen sowie eine höhere Alkoholkonzentration eingesetzt als in Schritt (x), um die Bindung der kurzkettigen Nukleinsäuren an das Trägermaterial zu fördern.

Gemäß einer Ausführungsform weist das erfindungsgemäße Verfahren wenigstens eines der folgenden Merkmale auf:
- dass die Konzentration der chaotropen Verbindungen in der Mischung in Schritt (x) und/oder Schritt (a) ≥ 2 mol/l, ≥ 2,4 mol/l oder ≥ 2,6 mol/l ist; und/oder
- dass die Konzentration der chaotropen Verbindungen in Schritt (a) ≥ der Konzentration der chaotropen Verbindungen in Schritt (x) ist; und/oder
- dass das nukleinsäurehaltige Ausgangsmaterial zuvor mit einem Lyse-Puffer behandelt wird; und/oder
- dass das nukleinsäurehaltige Ausgangsmaterial keine Zellen enthält und keine Zelllyse durchgeführt wird; und/oder
- dass das nukleinsäurehaltige Ausgangsmaterial zuvor mit einem Release-Puffer behandelt wird; und/oder
- dass keine Phenol-Extraktion durchgeführt wird; und/oder
- dass wenigstens 30% an kurzkettigen Nukleinsäuren mittels des Verfahrens isolierbar sind; und/oder
- dass wenigstens 50% an kurzkettigen Nukleinsäuren mittels des Verfahrens isolierbar sind; und/oder
- dass wenigstens 60% an kurzkettigen Nukleinsäuren mittels des Verfahrens isolierbar sind; und/oder
- dass eine wenigstens 2-fache Anreicherung der kurzkettigen Nukleinsäuren erzielt wird; und/oder
- dass eine wenigstens 5-fache Anreicherung der kurzkettigen Nukleinsäuren erzielt wird; und/oder
- dass eine wenigstens 10-fache Anreicherung der kurzkettigen Nukleinsäuren erzielt wird; und/oder
- dass kurzkettige Nukleinsäuren mit einer Länge isoliert und/oder angereichert werden, die ausgewählt sind aus der Gruppe von Nukleinsäuren mit einer Länge von ≤ 500bp, ≤ 400bp und/oder ≤ 300bp und/oder ≥ 50bp und/oder ≥ 100bp; und/oder
- extrazelluläre Nukleinsäuren isoliert werden.

Das nukleinsäurebindende Trägermaterial ist gemäß Anspruch 1 ausgewählt aus Silikamembrane und magnetische Partikel, die eine Silika- oder Glasoberfläche aufweisen. Entscheidend ist letztlich, dass das Trägermaterial in der Lage ist, Nukleinsäuren zu binden. Besonders bevorzugt werden Silikamaterialien eingesetzt. Hier haben sich sowohl der Einsatz von Silikamembranen als auch von magnetischen Partikeln bewährt, die eine Silika- oder Glasoberfläche aufweisen. Diese können im Wesentlichen perl- oder kugelförmig sein und weisen vorzugsweise eine Partikelgröße im Bereich von 0,02-30 µm, vorzugsweise 0,05-15 µm und besonders bevorzugt von 0,1-10 µm auf. Magnetische Silikapartikel, die vorteilhafter Weise im erfindungsgemäßen Verfahren eingesetzt werden können, sind z. B. in der internationalen Patentanmeldung WO 01/71732 beschrieben, worauf hiermit voll inhaltlich Bezug genommen wird.

Nach der Inkubation der Trägermatrix mit dem nukleinsäurehaltigen Material erfolgt die Abtrennung der Nukleinsäuren von der restlichen Probenflüssigkeit. Dies wird allgemein durch die Separation der erfindungsgemäß an die Partikel gebundenen Nukleinsäuren - bei Verwendung von magnetischen Silikapartikeln mit Hilfe eines Magnetfeldes - erreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden haben, entfernt werden. In Schritt (x) würde der Überstand weiterverarbeitet werden (die langkettigen Nukleinsäuren sind an die magnetischen Partikel gebunden); in Schritt a) werden die kurzkettigen Nukleinsäuren an die magnetischen Partikel gebunden und in Schritt b)ggf. nach Waschschritten optional von den Partikeln eluiert. Die Verarbeitung hängt von der Art des Gefäßes ab, in dem die Inkubation stattgefunden hat. Geeignete Verfahrensschritte zum Entfernen der Flüssigkeit sind z. B. abdekantieren, abpipettieren, abfiltrieren oder absaugen der Flüssigkeit.

Wie ausgeführt, können bspw. auch Silikamembranen eingesetzt werden. Flüssigkeiten können hier durch Zentrifugation oder Anlegung von Vakuum oder durch Druck entfernt werden. In vielen Anwendungen des erfindungsgemäßen Verfahrens, insbesondere bei der Aufreinigung von fötaler DNA aus maternalen Blutproben, werden die aufzureinigenden Nukleinsäuren in geringen Konzentrationen in großen Volumen vorliegen. Bei Einsatz einer Silikamembran besteht im Stand der Technik oft das Problem, dass große Probenvolumina die Membran verstopfen können. Nach dem erfindungsgemäßen Verfahren tritt dieses Problem jedoch nicht auf, da spezielle Puffersubstanzen/-konzentrationen eingesetzt werden. Hier ist es insbesondere vorteilhaft, dass Detergenzien eingesetzt werden.

Nukleinsäuren, die mit dem offenbarten Verfahren isoliert werden können, sind bspw. DNA, RNA, mRNA, mitochondriale, epigenetisch modifizierte, einzelsträngige, doppelsträngige, zirkuläre, Plasmid, Cosmid, artifizielle oder synthetische Nukleinsäuren, sowie cDNA und Fragmente davon. Das offenbarte Verfahren eignet sich insbesondere zur Anreicherung von kurzkettigen Nukleinsäuren (bspw. DNA und RNA in jeglicher Form, einschließlich nicht kodierender RNA wie bspw. miRNA oder synthetische Nukleinsäuren) mit einer Länge von ≤ 1000bp, ≤ 800bp, ≤ 500 bp, oder ≤ 300 bp. Gemäß einer offenbarten Ausführungsform werden kurzkettige Nukleinsäuren mit einer Länge isoliert und/oder angereichert, die ausgewählt sind aus der Gruppe von Nukleinsäuren mit einer Länge von ≤ 500bp, ≤ 400bp, ≤ 300bp und/oder ≥ 50bp, ≥ 100bp. Bevorzugt wird DNA und/oder RNA aufgereinigt. Besonders effizient lässt sich DNA oder RNA einer Länge ≥ 50 Nukleotiden aufreinigen. Zur Gewinnung von kurzkettiger RNA wird die aufgereinigte Nukleinsäure vorzugsweise mit DNase behandelt. Gemäß Anspruch 1 handelt es sich bei den Nukleinsäuren um kurzkettige, freizirkulierende DNA mit einer Länge von ≤ 500bp.

Die Größe der isolierten Nukleinsäuren kann auch durch Wahl der Alkoholkonzentration insbesondere in Kombination mit der Konzentration an chaotropen Agenzien variiert/gesteuert werden. Das erfindungsgemäße Verfahren ermöglicht in beiden Varianten (1-Schritt Verfahren und 2-Schritt Verfahren) eine effektive Isolierung auch von kurzkettigen Nukleinsäuren. Durch die geeignete Einstellung der Bindebedingungen wie hier beschrieben kann auch eine spezifische Anreicherung der kurzkettigen Nukleinsäuren erzielt werden. Das Verfahren eignet sich insbesondere zur Anreicherung von fötalen Nukleinsäuren, wie auch die nachfolgenden Beispiele belegen.

Das erfindungsgemäße Verfahren kann daher in vorteilhafter Weise eingesetzt werden, um extrazelluläre Nukleinsäuren zu isolieren, wie bspw. fötale DNA aus maternalem Blut zu isolieren. Mit der vorliegenden Erfindung wird daher auch ein Verfahren zur Anreicherung von fötalen Nukleinsäuren aus einer Blutprobe, insbesondere aus Blutplasma oder -serum, zur Verfügung gestellt, das dadurch gekennzeichnet ist, dass das erfindungsgemäße Verfahren zur Isolation/Aufreinigung von kurzkettigen Nukleinsäuren durchgeführt wird. Einzelheiten des Verfahrens sind oben dargelegt.

Weitere Anwendungsbereiche finden sich jedoch beispielsweise in der Forensik und in anderen Bereichen, wo die Aufreinigung kleiner Nukleinsäuren entscheidend ist. Darüber hinaus kann das erfindungsgemäße Verfahren auch im Rahmen der Diagnostik eingesetzt werden, um bspw. frei zirkulierende Tumor-Nukleinsäuren aus einer Probe, wie bspw. Blut aufzureinigen.

Mit der vorliegenden Erfindung wird ferner ein Verfahren zur Anreicherung von Nukleinsäuren aus einer Probe, außer zur Anreicherung von fötalen Nukleinsäuren aus einer Blutprobe, zur Verfügung gestellt, das dadurch gekennzeichnet ist, dass das erfindungsgemäße Verfahren zur Isolation/Aufreinigung von kurzkettigen Nukleinsäuren durchgeführt wird. Einzelheiten des Verfahrens sind oben dargelegt.

Das erfindungsgemäße Verfahren ist insbesondere für die Anwendung in der Diagnostik geeignet. Es ist auch automatisierbar und daher zur Anwendung auf entsprechenden Aufreinigungsrobotern einsetzbar.

Ferner wird ein Kit zur Isolierung und/oder Aufreinigung von Nukleinsäuren, insbesondere von kurzkettigen Nukleinsäuren, aus einem nukleinsäurehaltigen Ausgangsmaterial offenbart, aufweisend Puffer und/oder Reagenzien und optional wenigstens ein nukleinsäurebindendes Trägermaterial für die Durchführung des erfindungsgemäßen Verfahrens. Einzelheiten des Verfahrens sind oben dargelegt.

Ein entsprechender Kit kann zur Aufreinigung von fötaler DNA aus einer Blutprobe eingesetzt werden. Darüber hinaus wird ein entsprechender Kit zur Aufreinigung von kurzkettigen Nukleinsäuren aus einer Probe, außer zur Anreicherung von fötaler DNA aus einer Blutprobe offenbart.

### BEISPIELE

Die vorliegende Erfindung wird nunmehr anhand von Beispielen erläutert. Die durchgeführten Versuche erfolgten anhand der nachfolgend beschriebenen Versuchsvorschriften. Beispiele, die nicht von den Ansprüchen erfasst werden, sind Vergleichsbeispiele.

Ausgangspunkt für die Anwendung des erfindungsgemäßen Verfahrens zur Isolation von frei zirkulierender fötaler DNA aus maternalem Blut mit gleichzeitiger Anreicherung der fötalen DNA relativ zur maternalen DNA ist der in der Literatur beschriebene Befund, dass sich die durchschnittliche Länge von beiden Nukleinsäurespezies unterscheidet. Während man momentan davon ausgeht, dass die durchschnittliche Länge fötaler DNA kürzer als 500 bp ist, ist die durchschnittliche Länge maternaler DNA länger als 500 bp.

### Beispiel 1: Test zur Bestimmung der durchschnittlichen Länge fötaler und maternaler DNA

Drei verschiedene Plasma-Pools, die aus Blut von mit männlichen Föten schwangeren Frauen gebildet waren, wurden für die Untersuchung der Größenverteilung der sich darin befindlichen frei zirkulierenden DNA verwendet. Die Plasma-Pools waren Pool A, B und C. Pool A beinhaltete Plasma aus Blutproben von schwangeren Frauen im ersten bis dritten Trimester der Schwangerschaft. Pools B und C beinhalteten jeweils Plasma aus Blut, das den schwangeren Frauen im ersten und zweiten Trimester der Schwangerschaft abgenommen wurde; ein Zeitpunkt, der noch geringere Mengen frei zirkulierender fötaler DNA enthält, aber für den klinischen Diagnosezeitpunkt von größerer Relevanz ist.

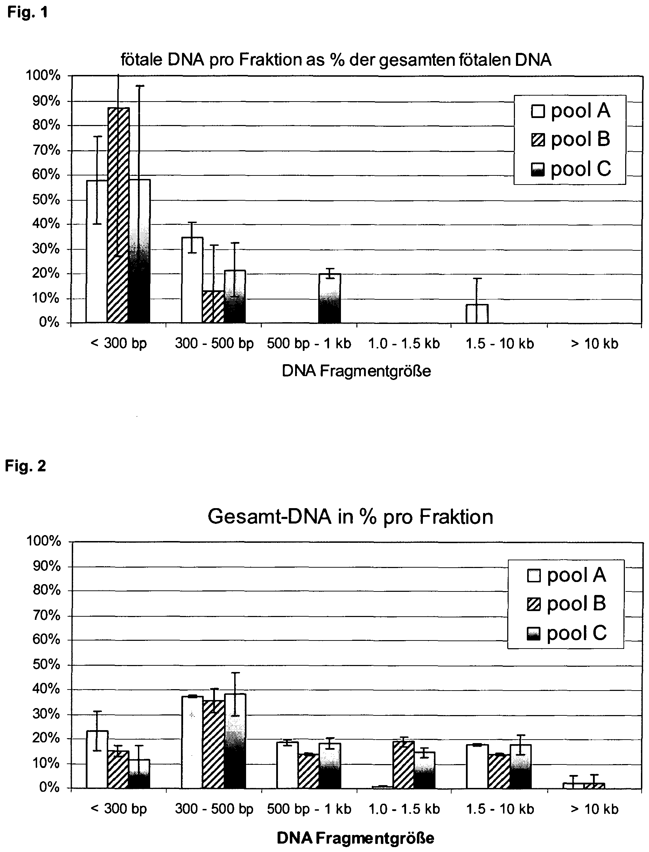

Als Ausgangsmaterial wurden 10ml Plasma und bei Pool A 5ml Plasma, verwendet. Vorgegangen wurde nach dem QIAamp Blood DNA Midi Protokoll (QIAGEN), welches auf ein Volumen von 10 ml angepasst wurde. Eluiert wurde jeweils mit 300 µl AE Puffer (QIAGEN, kommerziell erhältlich). Nach der Elution wurde eine Ethanol/Natriumacetat-Fällung durchgeführt und das getrocknete Pellet in 15 µl Puffer EB (QIAGEN, kommerziell erhältlich) aufgenommen. Nach einer Agarosegelelektrophorese wurden die einzelnen Größenfraktionen aus dem Gel herausgeschnitten und eine Gelextraktion nach dem QIAquick Vakuum-Protokoll zur Gelextraktion durchgeführt. Pro Gelfraktion wurde in 100 µl eluiert; in die nachfolgende PCR wurden jeweils 20 µl der Eluate in einer Doppelbestimmung eingesetzt. Amplifiziert wurde mit entsprechenden Primern zum einen der SRY Locus zum Nachweis der frei zirkulierenden fötalen DNA. SRY ist nur bei männlichen Individuen nachweisbar. Da ausschließlich Blut von Schwangeren verwendet wurde, die sicher einen männlichen Fötus austragen, waren alle SRY Signale auf DNA fötalen Ursprungs zurückzuführen. Amplifiziert wurde ferner mit entsprechenden Primern der c-myc Locus zum Nachweis der gesamten frei zirkulierenden DNA im maternalen Blut. Die Amplifikation wurde auf einem ABI 7500 Instrument durchgeführt (Applied Biosystems). Dabei ergab sich das in Fig. 1 und Fig. 2 gezeigte Ergebnis. Fig. 1 zeigt die Größenverteilung der fötalen DNA in Abhängigkeit der verwendeten Pools. Fig. 2 zeigt die Größenverteilung der Gesamt-DNA in Abhängigkeit der verwendeten Pools.

Dieses Experiment zeigt, dass, wie in der Literatur beschrieben, die fötale DNA nur in kurzen Fragmenten vorliegt. Dabei liegt der Hauptteil deutlich in der Fraktion kleiner 300 bp, ein signifikanter Teil zeigt Fragmentlängen von 300 bis 500 bp. Nur sehr wenig der frei zirkulierenden fötalen DNA im maternalen Blut ist länger als 500 bp. Auf der anderen Seite ist nicht alle maternale DNA größer als 500 bp. In etwa die Hälfte der maternalen DNA, die frei im Blut zirkuliert, ist ebenfalls nur 500 bp lang und kürzer, die andere Hälfte ist jedoch deutlich länger als 500 bp. Mittels einer Größenfraktionierung der isolierten/angereicherten Nukleinsäuren lässt sich demnach eine signifikante, relative Anreicherung fötaler DNA gegenüber maternaler DNA erreichen.

### Beispiel 2

Um die unterschiedliche Größenverteilung von fötaler DNA (der überwiegende Anteil ist kürzer als 300 bp, siehe Beispiel 1) und maternaler DNA (in der Hauptsache länger als 500 bp) zu simulieren, wurden in das Blutplasma als Hintergrund zwei unterschiedliche PCR-Amplifikate hinzugefügt. Ein 219 bp Fragment soll dabei die fötale DNA, ein Fragment von 1018 bp Länge die maternale DNA simulieren. In einem ersten Experiment wurden dazu relativ hohe Mengen dieser PCR-Amplifikate verwendet, nämlich jeweils 2x 10⁶ Kopien in 600 µl Plasma. Vorgegangen wurde dabei nach folgendem Protokoll:

Zu 600 µl Plasma in einem 5 ml Gefäß wurden 90 µl Protease (QIAGEN) und 600 µl Puffer AL (QIAGEN, kommerziell erhältlich), der Guanidin enthält, gegeben. Nach Mischen durch Vortexen wurde der Ansatz zur Lyse für 15 min bei 56 °C inkubiert. Nach der Lyse wurden die PCR Amplifikate von 219 oder 1018 bp Länge zum Lysat hinzugegeben. Die Bindebedingungen wurden mit 100 µl Isopropanol so eingestellt, dass dadurch in der Gesamtprobe eine Konzentration von 6,9 % (w/v) Isopropanol resultierte.

Nach Mischen durch Vortexen wurde für 10 min bei Raumtemperatur inkubiert. Zur Bindung wurden 50 µl MagAttract magnetische Partikel mit Silikaoberfläche (QIAGEN) hinzugegeben und der Ansatz für 5 min auf einem Schüttler binden gelassen. Nach der Bindung wurden die Partikel mittels eines Magneten vom Überstand separiert und der Überstand abgenommen. Der Überstand wurde bis zur weiteren Behandlung bei 4 °C aufbewahrt.

### Behandlung der MagAttract Partikel

Nach Abnahme des Überstandes wurden die Magnetpartikel mit 750 µl Puffer AW1 (QIAGEN, kommerziell erhältlich) für 5 min auf einem Plattenschüttler gemischt und anschließend die Partikelsuspension in ein 1,5 ml Reaktionsgefäß überführt. Nach magnetischer Separation in diesem Gefäß wurde der Überstand abgenommen und verworfen. Anschließend wurden die Partikel noch (jeweils nach 5 min Inkubation auf einem Schüttler) nacheinander mit 750 µl Puffer AW2 (QIAGEN, kommerziell erhältlich) und 750 µl Ethanol gewaschen. Nach dem Alkoholwaschschritt wurden die Partikel in einem Heizblock für 10 min bei 56 °C getrocknet. Zur Elution der an die Partikel gebundenen Nukleinsäuren wurden 200 µl RNase freies Wasser (QIAGEN) verwendet, wobei zur Elution erneut für 5 min geschüttelt wurde. Das Eluat wurde nach Magnetseparation in ein neues Gefäß überführt.

### Behandlung des Überstandes der Bindung

Der Überstand/Durchbruch der Bindung (das nicht an die Magnetpartikel gebundene Material) wurde wie folgt aufgearbeitet. Der Überstand nach der Bindung wurde mit 2 ml Puffer B6 (2,5 M GuHCl, 50% Isopropanol) versetzt, so dass eine Konzentration an Isopropanol von 32,4% (v/v) resultierte, durch Vortexen gemischt und 10 min bei Raumtemperatur inkubiert.

Nach der Inkubation wurden 50 µl MagAttract Partikel hinzugefügt und genau so vorgegangen, wie für den ersten Bindeansatz beschrieben (Waschen mit AW1 (QIAGEN, kommerziell erhältlich), AW2 (QIAGEN, kommerziell erhältlich), Ethanol, Elution in RNase freiem Wasser). Alternativ wurden zum Überstand nach der Bindung 2 ml 100% Isopropanol (Puffer B11) hinzugegeben, so dass eine Konzentrationen an Isopropanol von 61,9% (v/v) resultierte. Von den Eluaten wurden gleiche Aliquots abgenommen und mit Hilfe einer real-time PCR ein 119 bp Amplicon amplifiziert, das sowohl aus dem 219 bp Fragment als auch aus dem 1018 bp Fragment ein identisches Fragment ergibt. Der Nachweis des Amplicons erfolgte während der PCR mit Hilfe von SYBR Green.

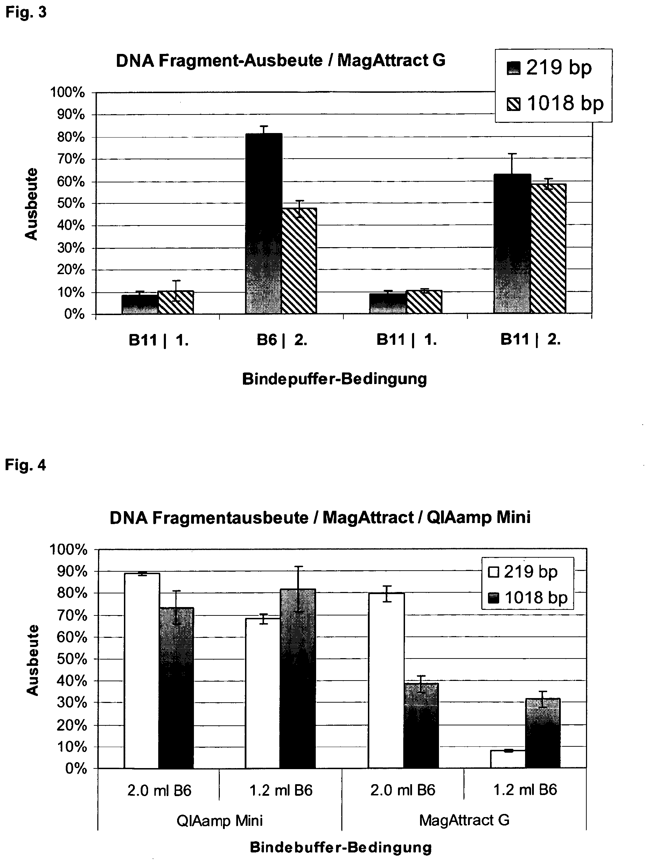

Dabei ergab sich das in Fig. 3 gezeigte Bild.

Dieses Experiment zeigt, dass sowohl vom 219 bp Fragment als auch vom 1018 bp Fragment unter den gewählten Bindebedingungen nur wenig Nukleinsäure an die ersten MagAttract Partikel bindet, jeweils etwa nur 10%. Bei neuer Einstellung der Bindebedingungen mit Puffer B6 bzw. B11 ergibt sich jedoch überraschenderweise ein Unterschied in der Ausbeute in Abhängigkeit der Fragmentgröße. Während mit Hilfe von Puffer B6 über 80% der kurzen DNA (die die fötale DNA repräsentiert) wiedergefunden werden können, ergibt sich für die längere DNA (die die maternale DNA repräsentiert) nur eine Ausbeute von etwa 50%. Beim Einsatz von Puffer B11 hingegen ergeben sich keine wesentlichen Unterschiede in der Ausbeute zwischen den beiden DNA-Fragmentlängen.

Dieser Versuch zeigt, dass mit Hilfe eines Zwei-Schritt-Bindesystems mit Hilfe von zwei festen Phasen unter geeigneten Bedingungen die fötale DNA relativ zur maternalen DNA angereichert werden kann, wobei nur sehr geringe Ausbeuteverluste an fötaler DNA zu verzeichnen sind.

### Beispiel 3

Vorgegangen wurde wie in Beispiel 2 beschrieben, nur dass diesmal nur 200.000 Kopien der definierten Fragmente eingesetzt wurden, um ein realistischeres Bild der tatsächlichen frei zirkulierenden Kopienzahlen im Blut zu simulieren. Gebunden an die Matrix unter der ersten Bedingung wurde diesmal mit 100 µl Puffer B11 (siehe oben) + 1,2 ml Puffer B6, so dass eine Konzentration an Isopropanol von 20,3% (v/v) resultierte. (siehe oben). Zur alternativen Bedingung für die Bindung an die Matrix wurden 100 µl Puffer B11 und 2,0 ml Puffer B6 zum Plasmalysat hinzugegeben. Zusätzlich wurden die DNA-Fragmente unter den beiden o.g. Pufferbedingungen im Bindeansatz jeweils an eine MagAttract Matrix bzw. QIAamp Mini Columns gebunden.

Dafür wurde wie folgt vorgegangen. Die Puffer B11 und B6 wurden zum Überstand gegeben, gemischt und für 10 min bei Raumtemperatur inkubiert. Das Lysat zweier Proben wurde vereinigt und mit Hilfe eines Extension Tubes (QIAGEN) unter Vakuum auf eine QIAamp Mini Column (QIAGEN) gegeben. Gewaschen wurde nacheinander mit 1000 µl (bei Bindung an MagAtrract Partikel) bzw. 750 µl (bei Bindung an QIAamp Mini Säulen) AW1 (QIAGEN, kommerziell erhältlich), AW2 (QIAGEN, kommerziell erhältlich) und Ethanol. Zum Trocknen wurden die Säulen 3 min bei 14.000 rpm zentrifugiert und die Säulen für 5 min in einen Heizblock bei 56 °C gestellt. Die MagAttract Partikel wurden wie in Beispiel 2 beschrieben behandelt). Eluiert wurde auch hier mit 200 µl RNase freiem Wasser (Zentrifugation für 1 min bei 14.000 rpm). Die nachfolgenden Real-time PCR ergab dabei das in Fig. 4 gezeigte Bild.

Dieser Versuch zeigt überraschenderweise, dass es bei gleicher Pufferzusammensetzung kaum einen Unterschied macht, ob als feste Phase magnetische Partikel oder eine Silikamembran eingesetzt werden. Bei Zugabe von 1.2 ml Puffer B6 werden längere DNA-Fragmente (ca. 1000 bp) in der erhaltenen DNA-Probe angereichert, während bei Zugabe von 2,0 ml Puffer B6 überraschenderweise umgekehrt kurze DNA-Fragmente (ca. 200 bp) angereichert werden. Bei Einsatz einer Silikamenbran (QIAamp Mini) ist die Ausbeute an DNA insgesamt höher als bei MagAttract Partikeln, allerdings ist auch die größenabhängige DNA-Bindung etwas weniger ausgeprägt. Eine Kombination von magnetischen Partikeln (erste Matrix) und Silikamembran (zweite Matrix) in einer zweistufigen DNA-Extraktion (bei der der DNA-haltige Überstand der 1. Bindung weiterverwendet und an eine Silikamembran gebunden wird) eignet sich folglich hervorragend, um kurze DNA-Fragmente in zwei Schritten effizient anzureichern und damit auch um fötale DNA relativ zu maternaler DNA auf der zweiten Matrix effektiv anzureichern.

### Beispiel 4

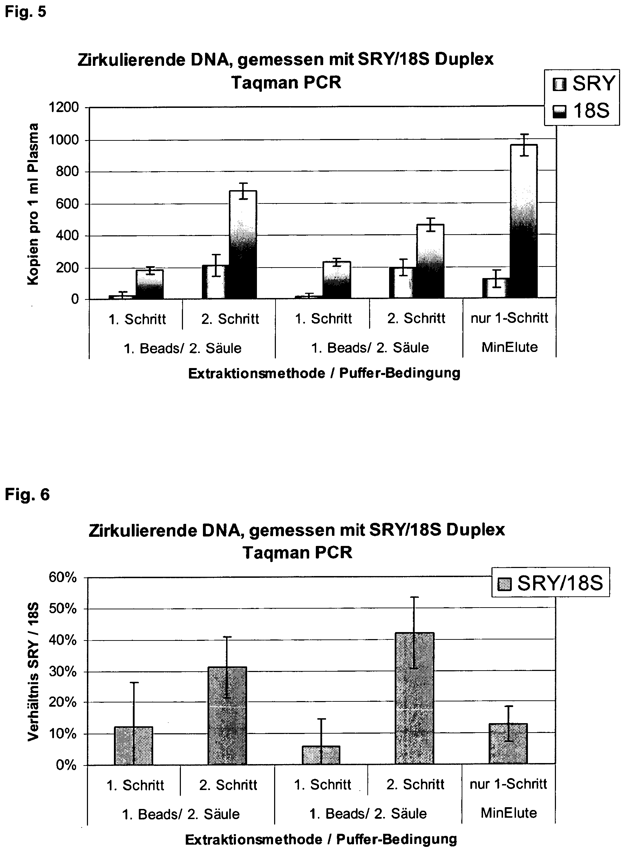

Vorgegangen wurde ähnlich wie in Beispiel 3 angegeben, nur dass diesmal mit einer realen Blutprobe gearbeitet wurde und ein zweistufiges Bindeverfahren eingesetzt wurde, Dabei wurde für den 1. Bindeschritt der Probe 1.2 ml Puffer B6 zugegeben, der Durchbruch bzw. Überstand des 1. Bindeschrittes wurde dann mit zusätzlichem Puffer B6 auf insgesamt 2,0 ml Puffer B6 eingestellt. Ausgangsmaterial war ein Pool aus Plasmaproben schwangerer Frauen des 1. und 2. Trimesters, von denen sicher war, dass sie einen Jungen austrugen. Zum Nachweis der fötalen DNA wurde in der nachfolgenden Real-time PCR der SRY-Locus amplifiziert, zum Nachweis der Gesamt-DNA der 18S Locus (siehe auch Beispiel 1). Zum Vergleich wurde ein 1-Säulen Protokoll nach dem QIAamp MinElute Virus Vacuum Protokoll durchgeführt, welches dem Stand der Technik entspricht. Die Ergebnisse sind in Fig. 5 gezeigt.

Das Ergebnis dieses Experimentes deckt sich mit dem Ergebnis von Beispiel 3, was demonstriert, dass das artifizielle System mit 219 bp und 1018 bp Fragment eine gute Simulation für die reale Situation (Beispiel 4) ist. Während durch die erste Matrix so gut wie keine fötale DNA verloren geht, binden signifikante Mengen der maternalen DNA bereits an die erste Matrix (hier: MagAttract magnetische Partikel). Maternale DNA wird daher effektiv in diesem Schritt (x) abgereichert und ist also während der Aufreinigung über die zweite Säule bereits depletiert. Im Vergleich zum Stand der Technik (MinElute 1-Schritt) bringt die Zwei-Matrix-Methode nicht nur eine erhöhte absolute Zahl an fötaler DNA, sondern bewirkt durch die zumindest teilweise Depletion der maternalen DNA durch die 1. Matrix auch ein deutlich besseres Verhältnis von fötaler zu maternaler DNA im Eluat, was die Nachweisbarkeit fötaler Erbsubstanz aus maternalem Blut deutlich voranbringt. Fig. 6 verdeutlicht noch einmal das verbesserte Verhältnis von fötaler zu maternaler DNA. Während sich bei der 1-Schritt Aufreinigung gemäß dem Stand der Technik ein Anteil von etwa 15% fötaler DNA im Eluat ergibt (quantifiziert über die SRY/18S duplex Real-time PCR), ist der Anteil bei einer 2-Schritt Aufreinigung mehr als verdoppelt mit einem Anteil von 30 bis 40%.

### Beispiel 5

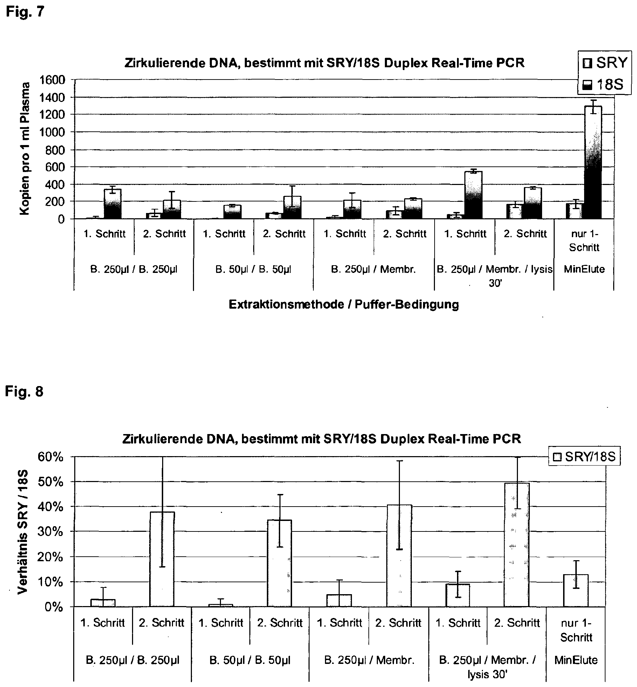

Vorgegangen wurde wie in Beispiel 4 beschrieben, nur dass diesmal ein höheres Volumen an Blutplasma verwendet wurde (3 ml pro Ansatz). Darüber hinaus wurden verschiedene Kombinationen von Bindungsoberflächen (MagAttract Partikel und QIAamp Mini Säulen) sowie verschiedene Mengen an MagAttract miteinander verglichen. Zusätzlich wurde beim Membran-2-Schritt-Protokoll für 30 min lysiert, nicht nur für 15 min. Die Ergebnisse sind in Fig. 7 gezeigt. Dieses Experiment bestätigt die Ergebnisse der vorangegangenen Experimente. Verglichen mit dem 1-Schritt-Protokoll des Standes der Technik ergeben sich mit dem 2-Schritt-Protokoll gemäß der Erfindung immer verbesserte Verhältnisse von fötaler DNA zu maternaler DNA und zwar unabhängig davon, ob im zweiten Bindeschritt magnetische Partikel mit Silikaoberfläche oder Silikamembrane eingesetzt werden. Eine verlängerte Lyse von 30 min scheint dabei noch zusätzlich das Verhältnis von fötaler DNA zu maternaler DNA zugunsten von fötaler DNA zu verbessern, so dass dadurch die maternale DNA deutlich depletiert werden kann. Diese Verbesserung des Verhältnisses ist auch in Fig. 8 gezeigt.

Während beim 1-Matrix Protokoll gemäß dem Stand der Technik sich nur ein Verhältnis von fötaler zu maternaler DNA von etwa 15% ergibt, lässt sich dieses Verhältnis durch das 2-Matrix-Protokoll auf bis zu 50% fötale DNA anheben. Der Anteil der fötalen DNA in der aufgereinigten zirkulierenden DNA aus maternalem Plasma kann also im Vergleich zum Stand der Technik deutlich gesteigert werden.

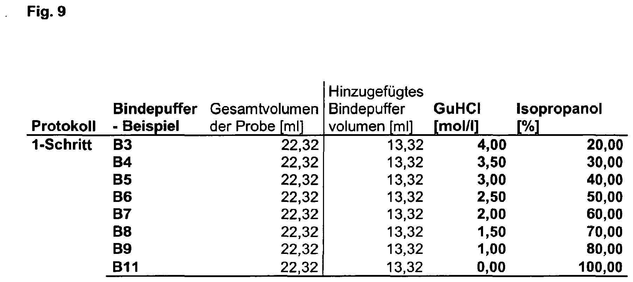

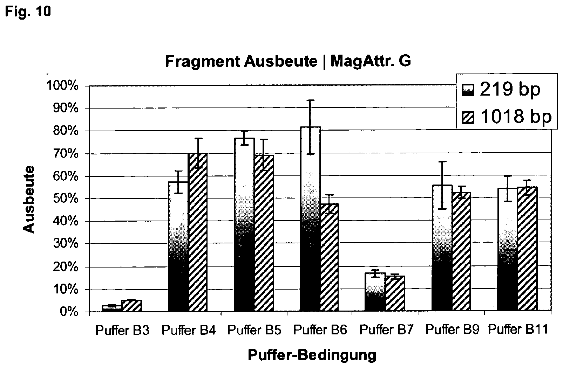

Die in Fig. 9 gezeigte Tabelle zeigt ferner verschiedene Reaktionsbedingungen in der Probe unter denen die Bindung der Nukleinsäuren an das Trägermaterial erfolgt (gemäß dem erfindungsgemäßen 1 - Schrittverfahren). Eingesetzt wurden MagAttract Partikel als Trägermaterial. Die Ergebnisse sind in Fig. 10 gezeigt.

### Beispiel 6

Um die Effizienz des erfindungsgemäßen Verfahrens zur Extraktion von frei zirkulierender DNA aus humanem Plasma zu vergleichen, wurden die folgenden Protokolle miteinander verglichen:
1. Das im Stand der Technik eingesetzte 1-Schritt Verfahren von frei zirkulierender DNA, nämlich das nachfolgend angegebene modifizierte QIAamp MinElute Virus Protokoll
2. Das 1-Schritt Protokoll gemäß der vorliegenden Erfindung zur Extraktion/Isolierung frei zirkulierender DNA ("one step").

Für diesen Versuch wurde gepooltes Plasma von männlichen Spendern in vier Extraktionswiederholungen pro Protokoll eingesetzt und getestet. Die DNA Extraktion erfolgte gemäß dem jeweiligen Protokoll. 5 ml Plasma wurde eingesetzt; die DNA wurde in 50 µl eluiert.

### 1. QIAamp MinElute Virus Vakuum Protokoll, modifiziert - Stand der Technik

Die frei zirkulierenden Nukleinsäuren wurden aus 5 ml EDTA Plasma isoliert. Das Protokoll erfolgte wie folgt:

### Release - Bedingungen

750 µl QIAGEN Protease (aufgelöst in Protease Solvent) wurde in ein 50 ml Gefäß pipettiert. Danach wurden 5 ml Plasma und 5 ml des Guanidin enthaltenden Puffers AL (mit 5,6 µg carrier RNA) zugegeben. Das Gefäß wurde verschlossen und gut gevortext, um eine homogene Lösung zu erhalten. Die homogene Lösung wurde dann 30 Minuten bei 56°C im Wasserbad inkubiert.

### Zugabe der Markerfragmente

Danach wurden zum homogenen Gemisch 20 µl einer Markerfragmentmischung hinzugegeben, um die Situation der fötalen Nukleinsäure gemischt mit maternalen Nukleinsäuren zu simulieren. Dazu wurden je 200.000 Kopien von 219 bp (entspricht der fötalen DNA) und 1.018 bp (entspricht der maternalen DNA) Fragmenten hinzugegeben.

### Bindung

6 ml Ethanol wurde zu dem Lysat hinzugegeben. Das Gemisch wurde gevortext und 5 Minuten auf Eis inkubiert. Das Lysat wurde auf eine QIAamp Minisäule geladen, wobei ein Extension-Tube auf einer QIAvac 24 Vakuumvorrichtung befestigt wurde. Das Lysat wurde durch Anlegung des Vakuums durch die Säule gezogen. Die Extension Tubes wurden vorsichtig entfernt.

### Waschschritte

600 µl des Puffers AW1 (QIAGEN, kommerziell erhältlich) wurde auf die Säule gegeben und Vakuum angelegt. Dieser Waschschritt wurde mit 750 µl des Puffers AW2 (QIAGEN, kommerziell erhältlich) und mit 750 µl Ethanol wiederholt.

Die Säulen wurden in 2 ml Collection-Tubes platziert, 3 Minuten bei 14.000 rpm zentrifugiert. Dann wurden die Säulen in frische Collection Tubes überführt und in einem Heizblock bei 56°C für 10 Minuten getrocknet.

### Elution

Die getrockneten Säulen wurden in 1,5 ml Gefäße platziert, und 50 µl des Puffers AVE (QIAGEN, kommerziell erhältlich) auf jede Säule gegeben; Inkubation für 3 Minuten und Zentrifugation für eine Minute bei 14.000 rpm.

Die so gewonnenen Nukleinsäuren befinden sich im Eluat.

### 2. Isolierung von frei zirkulierender Nukleinsäure gemäß dem erfindungsgemäßen 1-Schritt Verfahren

### Release

750 µl QIAGEN Protease (aufgelöst in Protease Solvent) wurde in ein 50 ml Gefäß pipettiert. 5 ml Plasma und 5 ml des Guanidin enthaltenden Lyse/Release Puffers AL (QIAGEN, kommerziell erhältlich, ohne carrier RNA) wurden hinzu gegeben.

Das Gefäß wurde verschlossen und gut gevortext, um eine homogene Lösung zu bilden. Die homogene Lösung wurde 30 Minuten bei 56°C im Wasserbad inkubiert.

### Zugabe der Markerfragmente

Auch hier wurden wiederum 20 µl einer Markerfragmentmischung hinzugegeben (je 200.000 Kopien an 200 bp und 1.000 bp Fragmenten, um das Verhältnis von fötaler zu maternaler DNA zu simulieren).

### Bindung

Danach wurden durch Bindepufferzugabe folgende Bindebedingungen eingestellt: ca. 25 bis 35% Isopropanol und mehr als 2M chaotrope Verbindungen. Entscheidend sind die Reaktionsbedingungen in der Gesamtprobe und nicht im Puffer, da die Reaktionsbedingungen im Gemisch entscheidend für die Bindungseffizienz der kurzkettigen Nukleinsäuren an das Trägermaterial sind.

Die Probe wurde gevortext und 5 Minuten auf Eis inkubiert.

Das mit dem Bindepuffer gemischte Lysat wurde dann auf eine QIAamp Minisäule mit Extension Tube geladen die auf einer QIAvac 24 Vakuumvorrichtung befestigt war. Das Lysat wurde durch die Säule durch Anlegen von Vakuum gezogen. Die Extension-Tubes wurden dann vorsichtig entfernt.

### Waschschritte

600 µl eines Waschpuffers, wie beispielsweise AW1 (QIAGEN, kommerziell erhältlich), wurde auf die Säule gegeben und mittels Vakuum abgezogen. Weitere Waschschritte mit 750 µl des Puffers AW2 (QIAGEN, kommerziell erhältlich) und mit 750 µl Ethanol können folgen.

Die gewaschenen Säulen wurden in 2 ml Collection-Tubes platziert und 3 Minuten bei 14.000 rpm zentrifugiert. Die Säulen wurden dann in frische Collection Tubes platziert und in einem Heizblock bei 56°C für 10 Minuten getrocknet.

### Elution

Die Säulen wurden in 1,5 ml Gefäßen platziert und 50 µl des Elutionspuffers AVE (QIAGEN, kommerziell erhältlich) wurde zu den Säulen gegeben, die Inkubation erfolgte für 3 Minuten, danach erfolgte ein Zentrifugationsschritt für eine Minute bei 14.000 rpm. Die überwiegend kurzkettigen Nukleinsäuren befinden sich im Eluat.

### 3. Ergebnisse

Die Ausbeute an DNA gemäß den einzelnen Protokollen wurde durch eine quantitative, duplexe, Real-Time PCR gemessen, wobei Taqman Proben eingesetzt wurden (4 PCR Wiederholungen pro DNA Extraktion). Die DNA wurde zum einen durch ein Y-chromosomales Target (DYS14) und ein 18S rDNA spezifisches Target bestimmt. Durch beide Methoden wurde letztlich die Konzentration der DNA in der Probe bestimmt. Da die 18S rDNA auf beiden Chromosomen vorhanden ist, liegt sie in doppelter Menge als das Y-chromosomale Target vor. Die DNA Ausbeute wurde als haploide Genom-Kopien pro ml Plasma angegeben.

Die Anreicherung von kleinen DNA Fragmenten wurde unabhängig bestimmt, in den angereicherten 200 bp und 1.000 bp DNA Fragmenten in einer duplexen, Real-Time PCR.

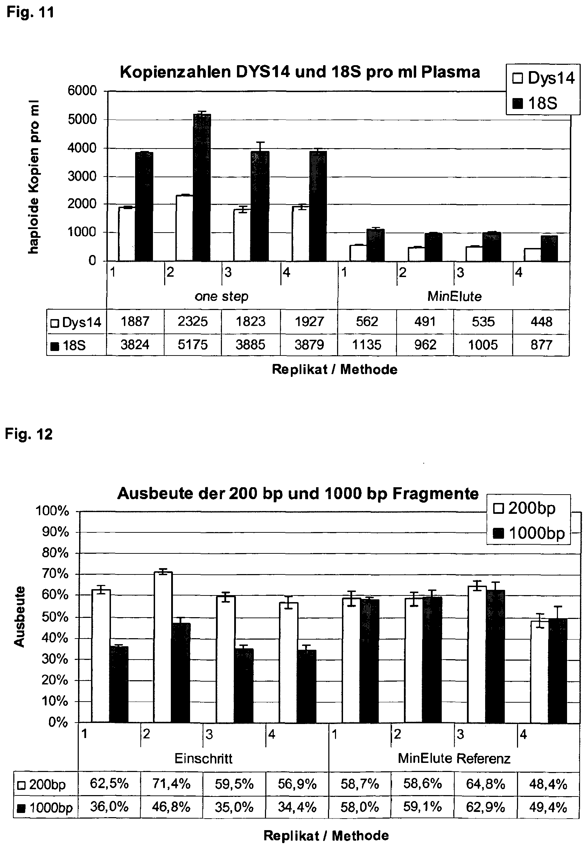

Die Ergebnisse sind in den Figuren 11 ff. dargestellt.

Figur 11 zeigt die DNA Ausbeute pro Probe (4 Proben wurden pro Extraktionsmethode getestet). Gezeigt ist daher der Gesamt-DNA Gehalt, der mittels der einzelnen Methoden erhalten wurde. Wie die Übersicht zeigt, wird mit dem erfindungsgemäßen 1-Schritt Verfahren eine deutlich höhere Ausbeute als mit dem im Stand der Technik bekannten 1-Schritt Verfahren (MinElute) erreicht. Die DNA Ausbeute liegt um ein Vielfaches höher, was grade zur Bestimmung von gering exprimierten/vorhandenen Nukleinsäuren von besonderem Vorteil ist.

Figur 12 zeigt die Ausbeute der eingespeisten 200 bp (simuliert die fötalen Nukleinsäuren) und 1.000 bp (simuliert die langkettigen maternalen Fragmente) DNA Fragmente. Wie oben dargelegt, wurden die 200 bp und die 1.000 bp Fragmente in einem Verhältnis von 1:1 eingespeist, nämlich je 200.000 Kopien. Die in Figur 12 gezeigte Grafik zeigt, ob bei den aufgereinigten Nukleinsäuren das Verhältnis von kurzkettigen zu langkettigen Nukleinsäuren nach wie vor 1:1 ist, oder ob eine Anreicherung der kurzkettigen Nukleinsäuren erzielt wurde. Wie Figur 12 zeigt, hat das 1-Schritt Verfahren des Standes der Technik (MinElute) ein Verhältnis von langkettigen zu kurzkettigen Nukleinsäuren von ca. 1:1. Es wird daher keine Anreicherung der kurzkettigen Nukleinsäuren erzielt. Unter der Fig. 12 ist auch noch tabellarisch gezeigt, wie viel der einzelnen Fragmente (200 bp und 1.000 bp) aus der Probe gewonnen wurde. So gibt beispielsweise die Angabe, dass 62,5% an 200 bp Fragmenten und 36% von 1.000 bp Fragmenten aufgereinigt wurden an, dass 62,5% der anfänglich eingespeisten 200.000 Kopien der 200 bp Fragmente aufgereinigt und damit isoliert wurden und 36% der 200.000 Kopien der 1.000 bp Fragmente. Wie gezeigt, wird mit dem erfindungsgemäßen 1-Schritt Verfahren eine deutliche Anreicherung der kurzkettigen Nukleinsäuren gegenüber der langkettigen Nukleinsäuren erzielt. Das Verhältnis ist nicht länger 1:1 (wie eingespeist), sondern es wurden deutlich mehr kurzkettige Nukleinsäuren aufgereinigt und damit im Eluat angereichert. Das erfindungsgemäße Verfahren ist daher dem Stand der Technik deutlich überlegen.

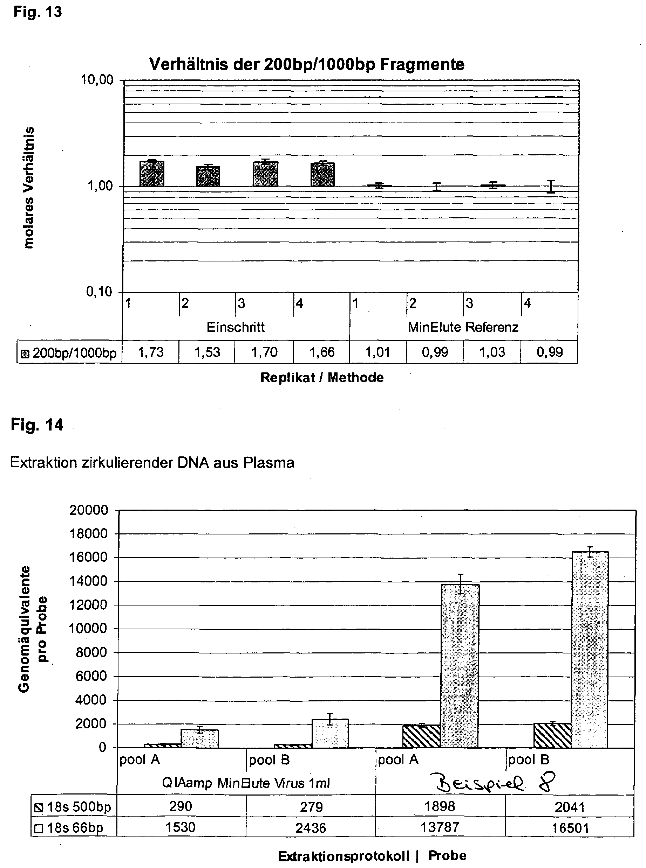

Das Verhältnis der aufgereinigten 200 bp zu 1.000 bp Fragmente ist zusätzlich in Figur 13 gezeigt. Während beim im Stand der Technik bekannten 1-Schritt Verfahren Werte von ungefähr 1 erzielt werden, ist das Verhältnis bei dem erfindungsgemäßen 1-Schritt Verfahren deutlich in Richtung der kurzkettigen Nukleinsäuren verschoben, die entsprechend bevorzugt angereichert/isoliert werden. Bei dem erfindungsgemäßen 2-Schritt Verfahren, bei dem die langkettigen Nukleinsäuren in einem Vorschritt zunächst abgereichert werden, wird sogar eine 5 bis 10-fache Anreicherung erzielt.

Wie die Ergebnisse zeigen, ist das erfindungsgemäße 1-Schritt Verfahren dem im Stand der Technik bekannten 1-Schritt Verfahren deutlich überlegen. Das erfindungsgemäße 1-Schritt Verfahren reichert kleine Nukleinsäuren während der Präparation an, was auf die einzigartigen Reaktionsbedingungen während der Bindung zurückzuführen ist, die zu einer bevorzugten Bindung von kurzkettigen Nukleinsäuren führt.

### Beispiel 7

Nachfolgend ist die Isolation von Nukleinsäuren, insbesondere zirkulierender RNA aus 5 ml Plasma oder Serum beschrieben.

1350µl Puffer AVE (QIAGEN, kommerziell erhältlich, enthält Guanidin) wird in ein Gefäß mit 1350µg lyophilisierter Carrier RNA gegeben, so dass eine Lösung von 1 µg/ml erhalten wird. Die Lösung von 1 µg/µl wird dann mit dem Puffer AL (QIAGEN, kommerziell erhältlich, enthält Guanidin) vermischt. Das Mischungsverhältnis wird je nach Anzahl der Proben angepasst. Für die Behandlung einer Probe werden 8,5 ml des Puffers AL mit 5,6 µl des Puffers AVE gemischt. Für mehr Proben müssen die Verhältnisse entsprechend angepasst werden. Das Gefäß wird zur Vermischung 10 Mal hin und her geschwenkt.

6 ml Protease Lösung (QIAGEN, kommerziell erhältlich) wird zu einer lyophilisierten QIAGEN Protease (7.5 A.U, kommerziell erhältlich) gegeben und vorsichtig gemischt.

500 µl QIAGEN Protease werden in ein 50 ml Gefäß (Tube) pipettiert und die 5 ml Plasma hinzu gegeben. Danach werden 8,5 ml des Puffers AL, gemischt mit Carrier RNA (siehe oben) hinzu gegeben und gevortext, um die Substanzen zu mischen.

Die gemischte Probe wird für 30 Minuten bei 56°C inkubiert.

7,5 ml eines Bindepuffers wird zu dem Lysat hinzu gegeben (enthält ca. 0,5 bis 1,5 mol/l Guanidinum, vorzugsweise um 1 mol/l und Isopropanol ca. 60 - 90 % (v/v); vorzugsweise mehr als 70%). Die Mischung wird für 30 Sekunden gevortext und 5 Minuten auf Eis inkubiert. Eine QIAamp Minisäule wird in einen VacConnector auf einem QIAvac 24 Plus inseriert und ein Extension Tube in der offenen QIAamp Minisäule platziert.

Das Lysat wird in das Extension Tube gefüllt und Vakuum angelegt. Sobald das Lysat durch die Säulen durchgezogen wurde, wird die Vakuumpumpe ausgestellt und der Druck ausgeglichen. Das Extension Tube wird verworfen.

Die Säule wird aus dem Vakuumträger entfernt und in ein 2 ml Auffanggefäß transferiert. Die Säule wird 1min bei 14000 rpm zentrifugiert.

Zur Präparation von RNA wird eine 10 µl DNAse I Stock Lösung zu 70 µl des Puffers RDD (QIAGEN, kommerziell erhältlich) pipettiert. Gemischt wird durch Schwenken des Tubes. Der Puffer RDD wird mit dem RNAse-free DNAse Set (QIAGEN, CAT.No.79254) geliefert.

Die Säulen werden wieder auf dem QIAvac 24 Plus Vakuumträger platziert. Die DNAse I Mischung wird direkt auf die QIAamp Mini Silikagelmembran pipettiert und bei gemäßigten Temperaturen (20 bis 30°C) für 15 Minuten inkubiert.

Danach werden 600µl des Puffers AW1 (QIAGEN, kommerziell erhältlich) auf die QIAamp Minisäule pipettiert. Danach wird Vakuum appliziert, um die Mischung durch die Säule zu ziehen. Im Anschluss werden 750 µl des Puffers AW2 (QIAGEN, kommerziell erhältlich) hinzu gegeben und durch Anlegen von Vakuum durch die Säule gezogen.

Im Anschluss werden 750 µl Ethanol (96-100%) auf die Säule gegeben und mittels Vakuum durchgezogen. Die QIAamp Minisäule wird im Anschluss aus dem Vakuumträger entfernt und der VacConnector wird verworfen. Die Säulen werden in ein sauberes 2 ml Auffanggefäß platziert und bei 20.000x g, 14.000 rpm für 3 Minuten zentrifugiert.

Die Säule wird in ein neues 2 ml Auffanggefäß platziert und bei geöffnetem Deckel für 10 Minuten bei 56°C getrocknet. Die QIAamp Minisäule wird dann in ein sauberes 1,5 ml Mikrozentrifugationsgefäß platziert und das Sammelgefäß wird verworfen. 20 bis 60 µl des Puffers AVE (QIAGEN, kommerziell erhältlich) wird auf die Mitte der QIAamp Minimembran pipettiert. Bei geschlossenem Deckel erfolgt eine Inkubation für 3min.

Im Anschluss erfolgt ein Zentrifugationsschritt bei 20.000 x g, 14.000 rpm für 1min um die RNA zu eluieren. Im Anschluss wird ein RNAse Inhibitor dazugegeben.

Mit dem entsprechenden Protokoll lässt sich kurzkettige RNA aufreinigen.

### Beispiel 8

Nachfolgend wird eine weitere bevorzugte Variante des 1-Schritt Verfahrens beschrieben, bei der Alkoholkonzentrationen von weniger als 25% (v/v) zum Einsatz kommen.

Diese Variante ist insbesondere zur Isolierung zirkulierender DNA und (m)RNA aus 5ml Plasma, Serum oder einer anderen zellfreien Körperflüssigkeit geeignet. Nachfolgend wurde dieses Verfahren genutzt, um zirkulierende Nukleinsäuren aus 5ml Plasma aufzureinigen (siehe Fig. 14).

### Lyse

Während der Lyse werden ca. 1,7 bis 2,2 mol/l Guanidiniumthiocyanat und 7,5 bis 9 (w/v) Detergenz eingesetzt.

Hierzu werden 500 µl QIAGEN Proteinase K in ein 50 ml Röhrchen pipettiert und 5 ml Plasma hinzugefügt. 4.0 ml ACL Puffer (QIAGEN, enthält 5.6 µg Carrier RNA) wird zugegeben, die Kappe wird verschlossen und durch Puls-vortexen für 30 s gemixt.

Die Probe wird auf 60°C erhitzt und für 30 min inkubiert. Das Röhrchen wird kurz geschleudert, um Tropfen aus der Innenseite des Deckels zu beseitigen

### Bindung

Während der Bindung wird zwischen 2,1 und 2,5 mol/l Guanidiniumthiocyanat, 9 bis 11 % (w/v) Detergenz sowie 19 bis 21 % (v/v) Isopropanol eingesetzt. Hierzu wurden 9.0 ml des Puffers ACB Puffers (QIAGEN) zum Lysat gegeben, der Deckel geschlossen und gründlich durch puls-vortexen für 15-30 s gemixt. Die Mischung wird für 5 min auf Eis inkubiert.

Für die Aufreinigung kann eine Säule (QIAamp Mini Column) eingesetzt werden. Die Säule wird in einen VacConnector platziert und ein 20 ml Verlängerungsrohr in die offene Säule plaziert. Dabei muss das Verlängerungsrohr fest in die Säule eingesetzt sein, um ein Verlust der Probe zu verhindern. Das Lysat von Schritt wird in das Verlängerungsröhrchen der Säule eingeführt und die Vakuum Pumpe angeschaltet. Wenn das komplette Lysat durch die Säule gezogen wurde, wird die Vakuum Pumpe abgestellt und der Druck auf 0 mbar reduziert. Das Verlängerungsröhrchen wird vorsichtig entfernt.

### Waschen

Zum Waschen werden 600µl Puffer ACW1 (QIAGEN) auf die Säule gegeben. Der Deckel der Säule wird offen gelassen und die Vakuumpumpe angeschaltet. Nachdem der ganze Puffer ACW1 durch die Säule gelaufen ist, wird die Vakuumpumpe ausgeschaltet und der Druck auf 0 mbar verringert.

750 µl Puffer des Waschpuffers ACW2 (QIAGEN) wird auf die Säule gegeben. Der Deckel der Säule wird offengelassen und die Vakuumpume angeschaltet. Nachdem der ganze Puffer ACW2 durch die Säule gelaufen ist, wird die Vakuumpume ausgeschaltet und der Druck auf 0 mbar verringert.

Danach werden 750 µl Ethanol (96-100%) auf die Säule gegeben. Der Deckel der Säule wird offengelassen und die Vakuumpumpe angeschaltet. Nachdem der ganze Ethanol durch die Säule gelaufen ist, wird die Vakuumpumpe ausgeschaltet und der Druck auf 0 mbar verringert.

Der Deckel der Säule wird verschlossen und die Säule in ein sauberes Sammelröhrchen platziert. Danach wird die Säule mit voller Geschwindigkeit (20,000 x g, 14,000 rpm) für 3 min zentrifugiert.

### Elution

Die Säule wird in ein neues 2 ml Sammelröhrchen plaziert, der Deckel geöffnet und die Verbindung bei 56°C für 10 min inkubiert, um die Membran komplett zu trocknen.

Die Säule wird in einem sauberen 1.5 ml Elutionsröhrchen platziert und das Sammelröhrchen entfernt. 20-150 µl Elutionspuffer (AVE Puffer, QIAGEN) wird auf das Zentrum der Membran der Säule aufgebracht. Der Deckel wird verschlossen und bei Raumtemperatur für 3 min inkubiert.

Bei voller Geschwindigkeit wird für 1 min zentrifugiert (20,000 x g; 14,000 rpm), um die Nukleinsäuren zu eluieren. Im Eluat sind sowohl zirkulierende DNA als auch RNA enthalten.

Die Ergebnisse der Aufreinigung gemäß dem Protokoll aus Beispiel 8 sind in Fig. 14 gezeigt. Frei zirkulierende zellfreie DNA wurde gemäß dem Protokoll nach Beispiel 8 (5 ml gepooltes Plasma) und dem QIAamp MinElute Virus Vakuum Kit (1 ml Plasma) als Referenz aufgereinigt. Das Elutionsvolumen betrug 100 µl. Die DNA Ausbeute wurde über eine Duplex Real-time PCR quantifiziert, wobei eine 500 bp und eine 66 bp lange Targetsequenz in der kodierenden Region für die 18S ribosomale RNA Verwendung fanden. Für die real-time PCR wurde der QuantiTect Multiplex PCR Kit verwendet. Die Nukleinsäureextratrionen wurde in 6 Replikaten pro Bedingung durchgeführt. Man sieht, dass das Protokoll gemäß Beispiel 8 eine höhere Ausbeute an zirkulierender DNA erzielt im Vergleich zu einer herkömmlichen, dem Stand der Technik entsprechenden Methode. Dabei ist die Ausbeute deutlich höher als allein aufgrund des höheren Probenvolumens zu erwarten wäre.

### Beispiel 9

Das Beispiel 9 zeigt ein bevorzugtes Verfahren zur Aufreinigung von RNA aus Proben, insbesondere Plasma, Serum oder anderen Körperflüssigkeiten. Die nachfolgend aufgeführten Konzentrationen sind für eine 5ml Probe ausgelegt.

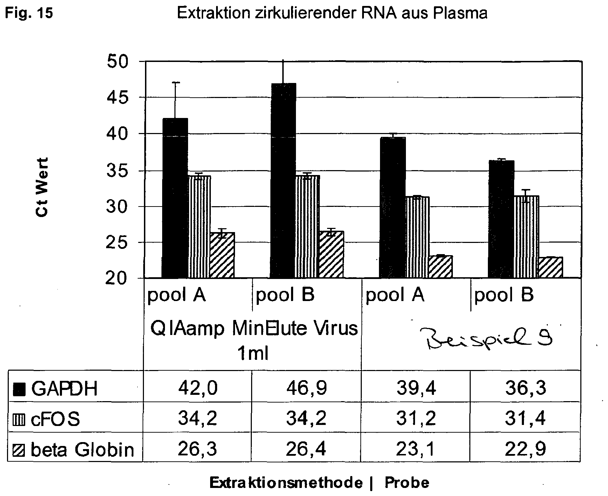

Das nachfolgende Protokoll wurde für die Aufreinigung von RNA aus 5ml Plasma eingesetzt (siehe Fig. 15).

Dabei wird das Verfahren wie in Beispiel 8 beschrieben durchgeführt. Jedoch werden zur selektiven Aufreinigung von RNA im Anschluss an die Bindung und vor der Durchführung der Waschschritte noch ein DNase Schritt durchgeführt, bei dem DNA durch Einsatz von DNase I verdaut wird:

Die Säule wird in ein 2ml Sammelröhrchen überführt und für 1min bei 14,000 rpm zentrifugiert. Durch diesen Schritt werden Lysatreste, die ggf. den DNase Verdau behindern könnten entfernt. Pro Probe werden 10µl DNAse Stocklösung zu 70µl Puffer RDD (QIAGEN) gegeben und durch invertieren der Probe gemischt.

Die Säulen werden wieder in ihre ursprünglichen Positionen gebracht. Der DNase I Inkubationsmix (80 µl) wird auf die Silica-gel Membran des Säulchens aufgebracht und bei gemäßigten Temperaturen (20-30°C) for 15 min inkubiert.

Im Anschluss wird wie im Beispiel 8 beschrieben gewaschen und eluiert.

Die Ergebnisse sind in Fig. 15 gezeigt. Frei zirkulierende zellfreie RNA wurden gemäß Beispiel 9 aufgereinigt (5 ml Plasma; einschließlich DNase-Behandlung der QIAamp-Säule It. Protokoll) und dem QIAamp MinElute Virus Vakuum Kit (1 ml Plasma) als Referenz aufgereinigt. Das Elutionsvolumen betrug 100 µl. Die RNA Ausbeute wurde über ein Real-time RT-PCRs quantifiziert, die spezifisch für GAPDH, c-fos, und beta-Globin mRNAs waren. Für die real-time RT-PCR wurde der QuantiTect Multiplex RT-PCR Kit verwendet. Die Nukleinsäureextratrionen wurde in 6 Replikaten pro Bedingung durchgeführt.

Man erkennt anhand der niedrigeren Ct-Werte ("cycle of threshold"), dass mit dem Verfahrensprotokoll gemäß Beispiel 9 eine höhere Ausbeute an zirkulierender mRNA erzielt im Vergleich zu einer herkömmlichen, dem Stand der Technik entsprechenden Methode. Dabei ist die Ausbeute deutlich höher als allein aufgrund des höheren Probenvolumens zu erwarten wäre.

## Patentansprüche

1. Verfahren zur Isolierung und/oder Aufreinigung von kurzkettigen Nukleinsäuren mit einer Länge von ≤ 500bp aus einem nukleinsäurehaltigen Ausgangsmaterial, **gekennzeichnet durch** die folgenden Verfahrensschritte:
(a) Bindung der Nukleinsäuren an ein nukleinsäurebindendes Trägermaterial, in dem man das Ausgangsmaterial in Gegenwart wenigstens einer chaotropen Verbindung und wenigstens eines verzweigten und/oder unverzweigten Alkohols, nämlich Isopropanol, mit dem nukleinsäurebindenden Trägermaterial in Kontakt bringt, wobei der Alkohol in einer Konzentration ≥ 15 % (v/v) und ≤ 25% (v/v) vorliegt und wobei das nukleinsäurebindende Trägermaterial eine nukleinsäurebindende Festphase ist, ausgewählt aus Silikamembrane und magnetische Partikel, die eine Silika- oder Glasoberfläche aufweisen, wobei während der Bindung nicht-ionisches Detergenz anwesend ist und die Konzentration der chaotropen Verbindung in Schritt a) bei ≥ 2 mol/l und ≤ 3,1 mol/l liegt;
(b) optional Elution der gebundenen Nukleinsäuren von dem nukleinsäurebindenden Trägermaterial;
wobei
- es sich bei den kurzkettigen Nukleinsäuren um kurzkettige, frei zirkulierende DNA handelt;
- das nukleinsäurehaltige Ausgangsmaterial aus Plasma und Serum ausgewählt ist; und
- Guanidiniumthiocyanat oder Guanidiniumisothiocyanat als chaotrope Verbindung eingesetzt wird.

2. Verfahren nach Anspruch 1, **gekennzeichnet durch** eines oder mehrere der folgenden Merkmale:
a. dass die wenigstens eine chaotrope Verbindung in Schritt (a) ein Thiocyanat ist; und/oder
b. dass wenigstens 30%, 50% und vorzugsweise wenigstens 60% der in dem Ausgangsmaterial vorhandenen kurzkettigen DNA mittels des Verfahrens isolierbar ist; und/oder
c. dass kurzkettige DNA mit einer Länge isoliert und/oder angereichert wird, die ausgewählt sind aus der Gruppe von DNA mit einer Länge von ≤ 400bp und/oder ≤ 300bp und/oder ≥ 50bp und/oder ≥ 100bp.

3. Verfahren nach Anspruch 1 oder 2, **dadurch gekennzeichnet, dass** magnetische Partikel, die eine Silika- oder Glasoberfläche aufweisen, als nukleinsäurebindendes Trägermaterial eingesetzt werden.

4. Verfahren nach einem oder mehreren der Ansprüche 1 bis 3, zur Isolierung und/oder Anreicherung von kurzkettiger, frei zirkulierender fötaler DNA aus Blutplasma oder - serum.

5. Verfahren gemäß einem oder mehreren der Ansprüche 1 bis 3 zur Isolierung und/oder Anreicherung von kurzkettiger, frei zirkulierender DNA aus einer Probe, außer zur Anreicherung von fötaler DNA aus Blutplasma oder -serum.

6. Verfahren nach einem oder mehreren der Ansprüche 1 bis 5, zur Isolierung und/oder Anreicherung von kurzkettiger, frei zirkulierender DNA aus Plasma.

7. Verfahren nach einem oder mehreren der Ansprüche 1 bis 6, zur Isolierung von kurzkettiger, frei zirkulierender DNA ausgewählt aus fötaler DNA und Tumor-DNA aus Plasma oder Serum.

8. Verfahren nach einem oder mehreren der Ansprüche 1 bis 7, **dadurch gekennzeichnet, dass** die Probe zunächst lysiert und/oder Nukleinsäuren freigesetzt werden.

9. Verfahren nach einem oder mehreren der Ansprüche 1 bis 8, **dadurch gekennzeichnet, dass** keine Phenol-Extraktion durchgeführt wird.

10. Verfahren nach einem oder mehreren der Ansprüche 1 bis 9, **dadurch gekennzeichnet, dass** die kurzkettige DNA doppelsträngig ist.

11. Verfahren nach Anspruch 1, **dadurch gekennzeichnet, dass** die chaotrope Verbindung Guanidiniumthiocyanat ist.

12. Verfahren nach Anspruch 1 oder 11, **dadurch gekennzeichnet, dass** der Alkohol in Schritt a) in einer Konzentration von 18 bis 20% (v/v) vorliegt.

13. Verfahren nach einem oder mehreren der Ansprüche 1 bis 12, **dadurch gekennzeichnet, dass** sich das nukleinsäurebindende Trägermaterial in einer Säule befindet.

14. Verfahren nach einem oder mehreren der Ansprüche 1 bis 13, **dadurch gekennzeichnet, dass** das nukleinsäurebindende Trägermaterial eine Silikamembran ist.

15. Verfahren nach einem oder mehreren der Ansprüche 1 bis 14 zur Anwendung in der Diagnostik.

## Claims

1. A method for isolation and/or purification of short chain nucleic acids having a length of ≤ 500bp from a nucleic acid containing starting material, **characterized by** the following method steps:
(a) binding the nucleic acids to a nucleic acid binding support material by contacting the starting material with the nucleic acid binding support material in the presence of at least one chaotropic compound and at least one branched and/or unbranched alcohol, namely isopropanol, wherein the alcohol is present in a concentration of ≥ 15% (v/v) and ≤ 25% (v/v), and wherein the nucleic acid binding support material is a nucleic acid binding solid phase, selected from silica membranes and magnetic particles having a silica or glass surface,
wherein during binding non-ionic detergent is present, and the concentration of the chaotropic compound in step a) is ≥ 2 mol/l and ≤ 3.1 mol/l;
(b) optionally eluting the bound nucleic acids from the nucleic acid binding support material;
wherein
- the short chain nucleic acids are short chain freely circulating DNA;
- the nucleic acid containing starting material is selected from plasma and serum; and
- guanidinium thiocyanate or guanidinium isothiocyanate is used as chaotropic compound.

2. The method according to claim 1, **characterized by** one or more of the following features:
a. that the at least one chaotropic compound in step (a) is a thiocyanate; and/or
b. that at least 30%, 50%, and preferably at least 60% of the short chain DNA present in the starting material can be isolated by means of the method; and/or
c. that short chain DNA with a length is isolated and/or enriched, which are selected from the group of DNA with a length of ≤ 400bp and/or ≤ 300bp and/or ≥ 50 bp and/or ≥ 100bp.

3. The method according to claim 1 or 2, **characterized in that** magnetic particles having a silica or glass surface are used as nucleic acid binding support material.

4. The method according to one or more of claims 1 to 3 for the isolation and/or enrichment of short chain freely circulating fetal DNA from blood plasma or serum.

5. The method according to one or more of claims 1 to 3 for the isolation and/or enrichment of short chain freely circulating DNA from a sample but not for the enrichment of fetal DNA from blood plasma or serum.

6. The method according to one or more of claims 1 to 5 for the isolation and/or enrichment of short chain freely circulating DNA from plasma.

7. The method according to one or more of claims 1 to 6 for the isolation of short chain freely circulating DNA selected from fetal DNA and tumor DNA from plasma or serum.

8. The method according to one or more of claims 1 to 7, **characterized in that** the sample is first lysed and/or nucleic acids are released.

9. The method according to one or more of claims 1 to 8, **characterized in that** no phenol extraction is performed.

10. The method according to one or more of claims 1 to 9, **characterized in that** the short chain DNA is double stranded.

11. The method of claim 1, **characterized in that** the chaotropic compound is guanidinium thiocyanate.

12. The method according to claim 1 or 11, **characterized in that** the alcohol in step a) is present at a concentration of 18 to 20% (v/v).

13. The method according to one or more of claims 1 to 12, **characterized in that** the nucleic acid binding support material is in a column.

14. The method according to one or more of claims 1 to 13, **characterized in that** the nucleic acid binding support material is a silica membrane.

15. The method according to one or more of claims 1 to 14 for use in diagnostics.

## Revendications

1. Procédé d'isolation et/ou de purification d'acides nucléiques à chaînes courtes avec une longueur de ≤ 1 000 paires de bases, à partir d'un matériel de départ contenant des acides nucléiques, **caractérisé par** les étapes de procédé suivantes :
(a) une liaison des acides nucléiques à un matériau de support se liant aux acides nucléiques, où le matériel de départ en présence d'au moins un agent chaotropique et d'au moins un alcool ramifié et/ou non ramifié, de préférence l'isopropanol, entre en contact avec le matériau de support se liant aux acides nucléiques, où l'alcool est présent selon une concentration ≥ 15 % (v/v) et ≤ 25 % (v/v) et où le matériau de support se liant aux acides nucléiques est une phase solide se liant aux acides nucléiques, choisie parmi une membrane de silice et des particules magnétiques, qui présentent une surface de silice ou de verre,
où pendant la liaison, un détergent anionique est présent et la concentration de l'agent chaotropique lors de l'étape a) est ≥ 2 mol/l et ≤ 3,5 mol/l ;
(b) une élution optionnelle des acides nucléiques liés à partir du matériau de support se liant aux acides nucléiques ;
où
- les acides nucléiques à chaînes courtes sont de l'ADN libre circulant ;
- le matériel de départ contenant des acides nucléiques est choisi parmi du plasma ou du sérum ; et
- l'agent chaotropique est le thiocyanate de guanidinium ou l'isothiocyanate de guanidinium.

2. Procédé selon une ou plusieurs des revendications 1 à 3, **caractérisé par** un ou plusieurs caractères distinctifs suivants :
a. le au moins un agent chaotropique lors de l'étape (a) est un thiocyanate ; et/ou
b. au moins 30 %, 50 % et de préférence au moins 60 % de l'ADN à chaînes courtes présent dans le matériel de départ est isolé grâce au procédé ; et/ou
c. l'ADN à chaînes courtes avec une certaine longueur est isolé et/ou enrichi, lesquels sont choisis parmi le groupe constitué d'ADN avec une longueur de ≤ 400 paires de bases et/ou de ≤ 300 paires de bases et/ou de ≥ 50 paires de bases et/ou de ≥ 100 paires de bases.

3. Procédé selon la revendication 1 ou 2, **caractérisé en ce que** les particules magnétiques qui présentent une surface de silice ou de verre sont utilisées comme matériau de support se liant aux acides nucléiques.

4. Procédé selon une ou plusieurs des revendications 1 à 3, pour une isolation et/ou un enrichissement d'ADN foetal libre circulant à chaînes courtes provenant de plasma ou de sérum sanguin.

5. Procédé selon une ou plusieurs des revendications 1 à 3, pour une isolation et/ou un enrichissement d'ADN libre circulant à chaînes courtes provenant d'un prélèvement, et pour un enrichissement d'ADN foetal provenant de plasma ou de sérum sanguin.

6. Procédé selon une ou plusieurs des revendications 1 à 5, pour une isolation et/ou un enrichissement d'ADN libre circulant à chaînes courtes provenant de plasma.

7. Procédé selon une ou plusieurs des revendications 1 à 6, pour une isolation d'ADN libre circulant à chaînes courtes choisi parmi un ADN foetal, et un ADN tumoral de plasma ou de sérum.

8. Procédé selon une ou plusieurs des revendications 1 à 7, **caractérisé en ce que** le prélèvement est tout d'abord lysé et/ou libère les acides nucléiques.

9. Procédé selon une ou plusieurs des revendications 1 à 8, **caractérisé en ce que** l'extraction a lieu sans phénol.

10. Procédé selon une ou plusieurs des revendications 1 à 9, **caractérisé en ce que** l'ADN à chaînes courtes est doubles brins.

11. Procédé selon la revendication 1, **caractérisé en ce que** l'agent chaotropique est le thiocyanate de guanidinium.

12. Procédé selon la revendication 1 ou 11, **caractérisé en ce que** l'alcool lors de l'étape a) est présent selon une concentration allant de 18 à 20 % (v/v).

13. Procédé selon une ou plusieurs des revendications 1 à 12, **caractérisé en ce que** le matériau de support se liant aux acides nucléiques est retrouvé dans une colonne de séparation.

14. Procédé selon une ou plusieurs des revendications 1 à 13, **caractérisé en ce que** le matériau de support se liant aux acides nucléiques est une membrane de silice.

15. Procédé selon une ou plusieurs des revendications 1 à 14, pour une utilisation lors du diagnostic.
